# EUROPEAN PATENT SPECIFICATION

(11) **EP 2 516 652 B1**
(45) Date of publication and mention of the grant of the patent: **05.11.2014**
(21) Application number: 10803285.5
(22) Date of filing: 20.12.2010
(51) Int. Cl.: C12N 15/82

(54) **Improved techniques for transfecting protoplasts**
Verbesserte Verfahren zur Transfektion von Protoplasten
Techniques améliorées de transfection de protoplastes

(30) Priority: 21.12.2009 US 288474 P; 24.12.2009 NL 2004020
(43) Date of publication of application: 31.10.2012
(60) Divisional application: 14184365.6
(73) Proprietor: Keygene N.V., 6700 AE Wageningen (NL)
(72) Inventor: BUNDOCK, Paul, NL-6700 AE Wageningen (NL); FIERENS-ONSTENK, Bernarda, Gerharda, Johanna, NL-6700 AE Wageningen (NL); LHUISSIER, Franck, NL-6700 AE Wageningen (NL)
(74) Representative: Raggers, René John
(86) International application number: PCT/NL2010/050872
(87) International publication number: WO 2011/078665

(56) References cited:
- WO-A1-2007/073166
- WO-A1-2010/074562
- WO-A2-01/68882
- WO-A2-2006/032504
- WO-A2-2006/134496
- WRIGHT D A ET AL: "High-frequency homologous recombination in plants mediated by zinc-finger nucleases", PLANT JOURNAL, BLACKWELL SCIENTIFIC PUBLICATIONS, OXFORD, GB, vol. 44, no. 4, 1 November 2005 (2005-11-01), pages 693-705, XP002403198, ISSN: 0960-7412
- CHAPEL M ET AL: "TEMPORARY INHIBITION OF CELL WALL SYNTHESIS IMPROVES THE TRANSIENT EXPRESSION OF THE GUS GENE IN BRASSICA-NAPUS MESOPHYLL PROTOPLASTS", PLANT CELL REPORTS, vol. 9, no. 2, 1990, pages 105-108, XP008133077, ISSN: 0721-7714
- TOWNSEND JEFFREY A ET AL: "High-frequency modification of plant genes using engineered zinc-finger nucleases.", NATURE 21 MAY 2009 LNKD- PUBMED:19404258, vol. 459, no. 7245, 21 May 2009 (2009-05-21), pages 442-445, XP002588972, ISSN: 1476-4687 cited in the application
- VAN PEER AREND F ET AL: "Phleomycin Increases Transformation Efficiency and Promotes Single Integrations in Schizophyllum commune", APPLIED AND ENVIRONMENTAL MICROBIOLOGY, vol. 75, no. 5, March 2009 (2009-03), pages 1243-1247, XP002588963, ISSN: 0099-2240
- AARTS MARIEKE ET AL: "Generation of a mouse mutant by oligonucleotide-mediated gene modification in ES cells", NUCLEIC ACIDS RESEARCH, OXFORD UNIVERSITY PRESS, SURREY, GB LNKD- DOI:10.1093/NAR/GKL896, vol. 34, no. 21, 1 December 2006 (2006-12-01), pages E147.1-E147.9, XP002573026, ISSN: 0305-1048 [retrieved on 2006-11-16]

### SEQUENCE LISTING

<110> Keygene NV
<120> Improved techniques for transfecting protoplasts
<130> P30039PC00
<150> US 61/288474
   <151> 2009-12-21
<150> NL2004020
   <151> 2009-12-24
<160> 9
<170> PatentIn version 3.3
<210> 1
   <211> 776
   <212> DNA
   <213> Lycopersicon esculentum
<220>
   <221> misc_feature
   <223> KU70
<400> 1
   ggaagatctg aacgaccagc ttaggaaacg catgtttaag aagcgcagag ttcgaagact      60
   tcgacttgta atttttaatg gattatctat cgaacttaac acctatgctt tgatccgtcc     120
   aactaatcca gggacaatta cttggcttga ttcgatgact aatcttcctt tgaagactga     180
   gagaaccttc atatgtgctg atactggtgc tatagttcag gagcctctaa aacgctttca     240
   gtcttacaaa aatgagaatg tcatcttttc tgcggatgag ctttcagaag tcaaaagagt     300
   ttcaactgga catcttcgtc tgttgggctt caagcctttg agctgcttaa aagactatca     360
   taacctgaag ccagcaactt ttgtctttcc cagtgatgag gaagtggttg gaagcacttg     420
   tcttttcgtt gctctccaaa gatcaatgtt gcggcttaag cgttttgcag ttgctttcta     480
   tgggaattta agtcatcctc aattggttgc tcttgttgca caagatgaag taatgactcc     540
   tagtggtcaa gtcgagccac cagggatgca tctgatttat cttccatatt ctgatgatat     600
   cagacatgtt gaagagcttc atactgatcc taattccgtg cctcatgcca ctgatgacca     660
   gataaagaag gcctccgctt tagtgagacg tattgacctc aaagattttt ctgtgtggca     720
   atttgctaat cctgcattgc agagacatta tgcagtatta caagctcttg cacttg         776
<210> 2
   <211> 884
   <212> DNA
   <213> Nicotiana benthamiana
<400> 2
   ggagctactg atagatcatt gattataatt gatgagttgg gccgtggtac atcaacctat      60
   gatggctttg gtttagcttg ggctatttgt gagcacattg ttgaagaaat taaggcacca     120
   acattgtttg ccactcactt tcatgagctg actgcattgg ccaacaagaa tggtaacaat     180
   ggacataagc aaaatgctgg gatagcaaat tttcatgttt ttgcacacat tgacccttct     240
   aatcgcaagc taactatgct ttacaaggtt caaccaggtg cttgtgatca gagttttggt     300
   attcatgttg ctgaatttgc aaattttcca ccgagtgttg tggccctggc cagagaaaag     360
   gcatctgagt tggaggattt ctctcctatt gccataattc caaatgacat taaagaggca     420
   gcttcaaaac ggaagagaga atttgaccct catgacgtgt ctagaggtac tgccagagct     480
   cggcaattct tacaggattt ctctcagttg ccactggata agatggatcc aagcgaggtc     540
   aggcaacagt tgagcaaaat gaaaaccgac ctggagaggg atgcagttga ctctcactgg     600
   tttcagcaat tcttttagtt cttcagatta gaactatatc ttctattctg tgaagcttgg     660
   gggaatgata gtgatgggtt ttgtggatat aacttagcct aagtgtaaag tttcgtttaa     720
   atccttaccc caaacatgat tctctgtaat caggggactt ttgtatgcat cctgtgttaa     780
   atagtaaacg ttatcttatg gtcagctaac attggtagta gtctattgaa ttattccttc     840
   acaacgacta aacaaccttc ccttctctta aaacacccta aact                      884
<210> 3
   <211> 18
   <212> DNA
   <213> artificial
<220>
   <223> primer
<400> 3
   accagcttag gaaacgca 18
<210> 4
   <211> 18
   <212> DNA
   <213> primer
<400> 4
   agcaccagta tcagcaca 18
<210> 5
   <211> 23
   <212> DNA
   <213> artificial
<220>
   <223> primer
<400> 5
   cacacattga cccttctaat cgc 23
<210> 6
   <211> 23
   <212> DNA
   <213> artificial
<220>
   <223> primer
<400> 6
   agaaatcctc caactcagat gcc 23
<210> 7
   <211> 786
   <212> DNA
   <213> Nicotiana benthamiana
<400> 7
   atgggaagag gatcgcatca ccaccatcat cataagcttc caaagaagaa gaggaaggtt     60
   ctcgagatgg tgagcaaggg ctaggagctg ttcaccgggg tggtgcccat cctggtcgag    120
   ctggacggcg acgtaaacgg ccacaagttc agcgtgtccg gcgagggcga gggcgatgcc    180
   acctacggca agctgaccct gaagttcatc tgcaccaccg gcaagctgcc cgtgccctgg    240
   cccaccctcg tgaccaccct gacctacggc gtgcagtgct tcagccgcta ccccgaccac    300
   atgaagcagc acgacttctt caagtccgcc atgcccgaag gctacgtcca ggagcgcacc    360
   atcttcttca aggacgacgg caactacaag acccgcgccg aggtgaagtt cgagggcgac    420
   accctggtga accgcatcga gctgaagggc atcgacttca aggaggacgg caacatcctg    480
   gggcacaagc tggagtacaa ctacaacagc cacaacgtct atatcatggc cgacaagcag    540
   aagaacggca tcaaggtgaa cttcaagatc cgccacaaca tcgaggacgg cagcgtgcag    600
   ctcgccgacc actaccagca gaacaccccc atcggcgacg gccccgtgct gctgcccgac    660
   aaccactacc tgagcaccca gtccgccctg agcaaagacc ccaacgagaa gcgcgatcac    720
   atggtcctgc tggagttcgt gaccgccgcc gggatcactc tcggcatgga cgagctgtac    780
   aagtaa                                                               786
<210> 8
   <211> 25
   <212> DNA
   <213> artificial
<220>
   <223> mutagenic oligonucleotide
<220>
   <221> modified_base
   <222> (1) .. (4)
   <223> phosphorothioate modified nucleotides
<220>
   <221> modified_base
   <222> (22) .. (25)
   <223> phosphorothioate modified nucleotides
<400> 8
   tgaacagctc ctcgcccttg ctcac 25
<210> 9
   <211> 25
   <212> DNA
   <213> artificial
<220>
   <223> mutagenic oligonucleotide
<220>
   <221> modified_base
   <222> (1)..(4)
   <223> phophorothioate modified nucleotides
<220>
   <221> modified_base
   <222> (22)..(25)
   <223> phophorothioate modified nucleotides
<400> 9
   tgaacagctc ctagcccttg ctcac 25

## Description

### Field of the Invention

The present invention relates to methods for the introduction of foreign molecules of interest in plant cell protoplasts. The disclosure further relates to transfected plant cell protoplasts and to kits for carrying out the method.

### Background of the invention

Genetic modification is the process of deliberately creating changes in the genetic material of living cells with the purpose of modifying one or more genetically encoded biological properties of that cell, or of the organism of which the cell forms part or into which it can regenerate. These changes can take the form of deletion of parts of the genetic material, addition of exogenous genetic material, or changes like substitutions in the existing nucleotide sequence of the genetic material.

Methods for the genetic modification of eukaryotic organisms have been known for over 20 years, and have found widespread application in plant and animal cells and microorganisms for improvements in the fields of agriculture, human health, food quality and environmental protection.

The common methods of genetic modification consist of adding exogenous DNA fragments to the genome of a cell, which will then confer a new property to that cell or its organism over and above the properties encoded by already existing genes (including applications in which the expression of existing genes will thereby be suppressed). Although many such examples are effective in obtaining the desired properties, these methods are nevertheless not very precise, because there is no control over the genomic positions in which the exogenous DNA fragments are inserted (and hence over the ultimate levels of expression), and because the desired effect will have to manifest itself over the natural properties encoded by the original and well-balanced genome. A common problem encountered is that due to random integration of the exogenous DNA fragments in the genomic DNA of the host essential or beneficial genes are inactivated of modified, causing unwanted loss of desirable characteristics of the host.

On the contrary, methods of genetic modification that will result in the addition, deletion or conversion of nucleotides in predefined genomic loci will allow the precise modification of existing genes.

With the advent of genomics over the past decade, it is now possible to decipher the genomes of animals, plants and bacteria quickly and cost effectively. This has resulted in a wealth of genes and regulatory sequences that can be linked to phenotypes such as disease susceptibility in animals or yield characteristics in plants. This will allow the putative function of a sequence to be quickly established, but the ultimate proof that a gene is responsible for an observed phenotype must be obtained by creating a mutant line which shows the expected altered phenotype.

Unlike animal's, plant cells are surrounded by a thick cell wall composed of a mixture of polysaccharides and proteins, and while animal cells are readily amenable to the introduction of foreign molecules, plant cells are more recalcitrant and require somewhat more invasive methods. The prior art procedures to introduce foreign molecules into a plant cell can be divided in 2 categories.

The first category regroups all methods making use of mechanical introduction of the molecule of interest into the plant cell by puncturing the plant cell wall. This can be achieved by biolistics delivery for which the molecule of interest is coated onto metal beads, gold or tungsten, which are propelled into the cell using a gas-pressurized device. The efficiency of such an approach is however, rather low and since not all cells are transformed, selection is required which restricts the number of targets. Another approach uses micro- or nano-needles connected to a micro-manipulator to inject the compound directly into the plant cell through the cell wall. However, micro-injection requires specialized equipment and a significant amount of skill. The method is also tedious and time consuming and offers little advantages over biolistics delivery. Yet another method makes use of carbon nanotubes containing the molecule of interest and whose extremities are coated with cell wall digesting enzymes. The nanotubes will supposedly locally degrade the cell wall and puncture the plasmalema allowing the delivery of their content into the host cell. While being less invasive than micro-injection or biolistics bombardment, the limitations described above also apply here.

The second category regroups all methods in which the entire plant cell wall is enzymatically removed prior to the introduction of the molecule of interest. The complete removal of the cell wall disrupts the connection between cells producing a homogenous suspension of individualized cells which allows more uniform and large scale transfection experiments. This comprises, but is not restricted to protoplast fusion, electroporation, liposome-mediated transfection, and polyethylene glycol-mediated transfection. Protoplast preparation is therefore a very reliable and inexpensive method to produce millions of cells and is often preferred over other methods for its flexibility, efficiency and yield.

Protoplasts can be isolated from almost every plant tissue. The primary source of protoplasts is mesophyll tissue which yields high amounts of protoplasts per gram of fresh weight. The use of other types of tissue mostly depends on the availability of existing procedure for the system under consideration and the end goal of the experiment.

Many biological processes, if not all, are spatially and timely regulated. A cell has its own biological clock of which the cell cycle is the most obvious representation. Every single cell will go trough a series of developmental stages such as growth (G0, G2), DNA replication (S), division (M) and quiescence (GO). It is therefore of relevance to address the state of the system under consideration when designing experiments meant to interact with specific pathways. The introduction of the molecule of interest has to be carefully timed in order to match the process studied. The molecule of interest either has to be stable in the cellular environment over a long period of time until it can perform its action or has to be delivered shortly before the process under investigation begins. For instance, in studies of microtubule dynamics during pre-prophase band formation by introduction of labelled tubulin in the cell, one has to make sure that tubulin is delivered shortly before pre-prophase band formation unless labelled tubulin is sufficiently stable to withstand enzymatic degradation until pre-prophase band formation starts. For that particular example, another consideration would be the incorporation of the fluorescent tubulin in structures other than the pre-prophase band, hence the need to deliver the probe at the desired time.

Unfortunately, except for the rare cases of cell suspension cultures, mesophyll cells from which protoplasts can be derived are in a quiescent state (GO) and only when the protoplasts are triggered with a proper hormone balance will they re-enter the cell cycle and actively start streaming. The time needed for one quiescent protoplast to go through one round of cell cycle greatly varies from system to system and can take from a few hours to several days. Furthermore, as soon as the enzyme mixture used to generate the protoplasts is washed away, the protoplasts will start reforming their cell wall, which will reduce or even completely preclude the introduction of foreign molecules if precautions are not taken to slow down or prevent cell wall reformation. Protoplasts therefore cannot just be left unattended until they reach the appropriate stage when the molecule of interest is to be delivered, cell wall reformation has to be actively prevented while the streaming capacity of the protoplasts should be retained.

### Summary of the invention

The invention is as defined in the accompanying claims. The present inventors have set out to overcome these disadvantages in the art and have devised a method in which protoplasts and cell cycles can be controlled and transfected more efficiently and in a more controllable manner.

The present inventors have now found that a combination of two transfection steps allows the detailed control over several biological processes in the protoplasts. The combination of two transfection steps may be combined with the use of cell wall inhibitors, and/or a synchronization step of the cell phase. The inventors have found that introduction of various compositions that in a first transfection step interact with certain pathways and/or introduces double strand DNA breaks and a second step in which the transfection with the foreign molecule is performed allows for improved efficiency and control over transfections processes. The present inventors have further found that by adding one or more non-enzymatic chemical compounds to the protoplasts, which chemical compound(s) interfere with cell wall formation such as by inhibiting cellulose synthase, cellulose deposition or capturing nascent cellulose microfibrils, the timing and efficiency of the introduction of foreign molecules can be enhanced and optimised through the possibility of delivery of the foreign molecules closer in time to the desired phase in the cell cycle. The present inventors have also found that by synchronizing the cells in a certain cell phase, increased transfection can be achieved.

In broader terms, the (transient) suppression of the Mismatch Repair System and/or the NHEJ pathway and/or the introduction of DNA double strand breaks and (ii) the transfection of the protoplast with a foreign molecule of interest such as a mutagenic oligonucleotide, optionally combined with transient inhibition of cell wall reformation in protoplast systems and/or synchronization of the cell cycle phase is extremely valuable when a cell system has to be transfected at a specific stage of the cell cycle when the cells become proficient in certain biological/biochemical processes that are timely distant from the point of protoplast isolation. Furthermore, the transient inhibition of cell wall reformation in protoplast systems allows the sequential introduction of transiently expressed plasmids, which combined action leads to the desired outcome. For instance, gene targeting is more efficient if the ZFN construct is introduced some time, for example, 4, 6, 12, 18 or 24 hours before the donor construct is introduced. This allows the ZFNs to be expressed and induce the DSBs necessary for proper gene targeting events to take place.

### Detailed description

The invention is as defined in the accompanying claims. In a first aspect, the disclosure relates to a method for the introduction of one or more molecules of interest in a plant cell protoplast comprising the steps of
- providing the plant cell protoplast by enzymatically degrading and/or removing the cell wall from a plant cell;
- performing a first transfection of the plant cell protoplast with
   i. a first composition that is capable of altering the regulation of one or more pathways selected from the group consisting of Mismatch Repair System, Non-Homologous End Joining; and/or
   ii. a second composition that is capable of inducing a DNA double strand break
- performing a second transfection of the plant cell protoplast with one or more molecules of interest;
- allowing the cell wall to form;
wherein the second transfection is performed after the first transfection.

It will be understood by the skilled person that the term "and/or" implies within the context of the current disclosure that either a transfection with the first composition, or a transfection with the second compositions, or a transfection with both can be performed. So the first transfection according to the current disclosure, and in all it embodiments may comprise a first composition or a second composition or both.

In the first step of the method, protoplasts are provided from plant cells. The protoplasts can be provided using the common procedures (e.g. using macerase) using for the generation of plant cell protoplasts. Plant cell protoplasts systems have thus far been described for tomato (Solanum Lycopersicon), tobacco (Nicotiana tabaccum) and many more (Brassica napus, Daucus carota, Lactucca sativa, Zea mays, Nicotiana benthamiana, Petunia hybrida, Solanum tuberosum, Oryza sativa). The present invention is generally applicable to any protoplast system, including those, but not limited to, listed herein.

The protoplast can be derived form mesophyllic cells (not actively dividing, from meristem cultures (actively dividing) and from cell suspension (actively dividing)

The protoplast can be transfected with a first composition that is capable of altering the regulation of one or more of the pathways selected from the group consisting of the Mismatch Repair system, the Non-homologous End-Joining pathway. Preferably the transfection is transient. Preferably the Mismatch Repair system, the Non-homologous End-Joining pathway are down-regulated.

The regulation of the pathways is preferably achieved through the use of dsRNAs that are capable of regulating these pathways. Examples and guidance for the selection and design of the appropriate compositions are provided herein below. In one embodiment, the first composition is capable of altering the regulation of one or more of MutS, MutL, MutH, MSH2, MSH3, MSH6, MSH7, MLH1, MLH2, MLH3, PMS1, the DNA-PK complex Ku70, Ku80, Ku86, Mre11, Rad50, RAD51, XRCC4, Nbs1.

### Mismatch repair system

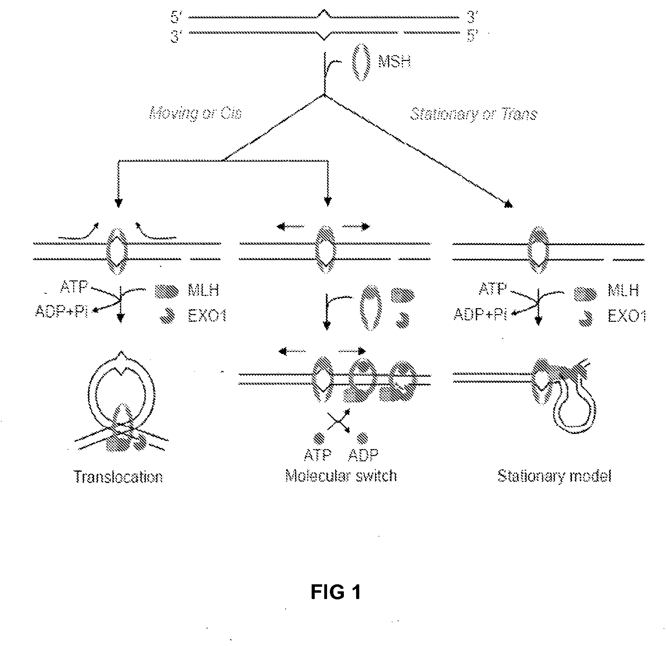

Many lesions are repaired by the so-called mismatch repair system (MMR). In E. Coli, the MMR consists of 3 major complexes, MutS, MutL and MutH. MutS is involved in the recognition of the mismatch and signaling towards the second complex MutL which recruits MutH. MutH possesses a nicking activity that will introduce a nick in the newly synthesized DNA strand containing the mismatch. The presence of a nick in the newly synthesized strand signals to an exonuclease the stretch of DNA to be degraded, including the mismatch nucleotide. A DNA polymerase will then fill-in the gap in the daughter strand. Orthologs of E. Coli MMR genes, except for MutH whose function is carried out by MutL, can be found in all eukaryotes (for review see Kolodner & Marsishky 1999, Curr.Opin.Genet-Dev. 9: 89-96). In plants, four MutS orthologs (MSH2, MSH3, MSH6 and MSH7) and four MutL orthologs (MLH1, MLH2, MLH3 and PMS1) are present. Mismatch recognition of base-base mispairs or single extrahelical nucleotides is accomplished by MutSα (a MSH2::MSH6 heterodimer) while larger extrahelical loopouts are recognized by MutSβ (MSH2::MSH3 heterodimer). The MSH7 gene has been identified in plants but not thus far in animals. MSH7 is most similar to MSH6 and also forms a heterodimer (MutSy) with MSH2 (Culligan & Hays, 2000, Plant Cell 12: 991-1002). The MMR pathway is illustrated in Fig 1, taken from Li, 2008 Cell Research 18:85-98.

Recently, a method for transient suppression of specific mRNA in plant protoplasts has been proposed (An et al. 2003 Biosci.Biotechnol.Biochem. 67: 2674-2677) and it was now found that this is a valuable tool for transient suppression of (endogenous) MMR genes in plants.

Sequences from genes associated with the MMR pathway (such as MSH2, MSH3, MSH6, MSH7, MLH1, MLH2, MLH3 and PMS1) that can be used in the compositions used to alter the regulation of the pathway, such as the generation of the dsRNA are available from Public databases such as GenBank entry AF002706.1 for AtMSH2 and described herein elsewhere. The desired plant specific sequences can be identified by designing primers based on , for instance available Arabidopsis sequences, and subsequently identifying the desired orthologs.

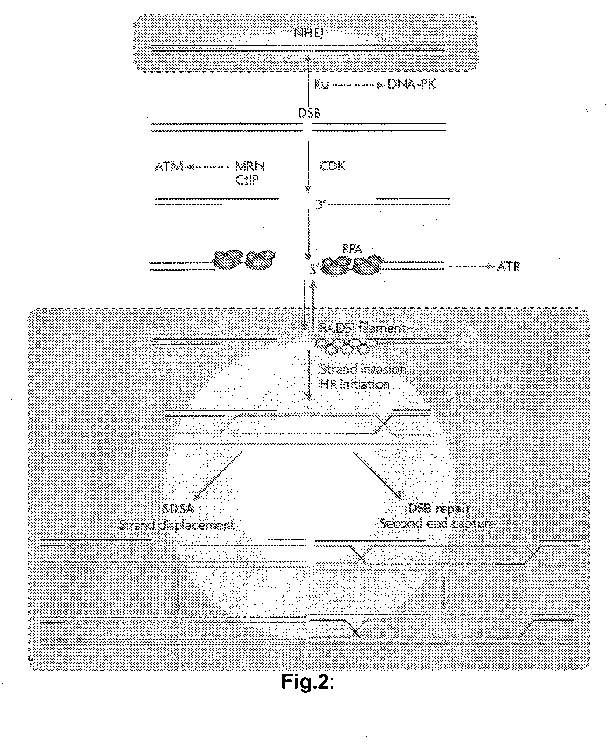

The most toxic lesions are DNA double strand breaks (DSB). DSB can result from the action of endogenous or exogenous genotoxic agents, such as reactive oxygen species - especially the hydroxyl radical - ionizing radiation or chemicals (including chemotherapeutic agents used for the treatment of cancers). Cellular processes such as the repair of other kinds of DNA lesions, or DNA replication also give rise to DSB. For example, DNA repair by nucleotide- or base-excision repair involves endonucleases, which introduce single-strand nicks. The co-incidence of single-strand nicks or gaps on the two DNA strands leads to the formation of a DSB. In a similar way, a single-strand nick or gap upstream of a replication fork can be processed into a DSB by unwinding of the DNA double helix (Bleuyard et al., 2006, DNA repair 5:1-12). Two competitive pathways (Figure 2, From Branzei and Foiani, 2008 - 8(9):1038-46) exist to repair DSBs, namely non-homologous end joining (NHEJ) and homologous recombination (HR). Double strand breaks (DSBs) are repaired preferably by non-homologous end joining (NHEJ) during G1 phase and by homologous recombination (HR) during S and G2 phases of the cell cycle. Binding of the Ku heterodimer to DSBs triggers the recruitment of DNA-PK catalytic subunit and sealing of the DSBs by NHEJ. By contrast, DSBs that occur during S and G2 phases preferential activate ATM, through the MRE111-RAD50-NBS1 complex. The higher cyclin dependent kinase (CDK) activity that is specific for S and G2 phase of the cell cycle promotes DSB resection, exposing 3'overhangs of single stranded DNA (ssDNA). When the ssDNA of 3' overhangs is coated with replication protein A (RPA), it activates ATR; RPA can be removed and replaced by RAD51 with the help of mediator protein such as RAD52. This leads to the formation of RAD51 presynaptic filaments, which initiate HR by invading the homologous region in the duplex to forma a DNA joint called a D-loop which can be further extended by DNA synthesis. Strand displacement of this intermediate by a DNA helicase channels the reaction towards synthesis-dependent strand annealing (SDSA). Alternatively the second DSB end can be captured giving rise to a double Holliday junction intermediate which can be resolved by endonuclease or dissolved by the combined action of a helicase (BLM) and a topoisomerase (TOP3).

### Non-homologous End-Joining pathway

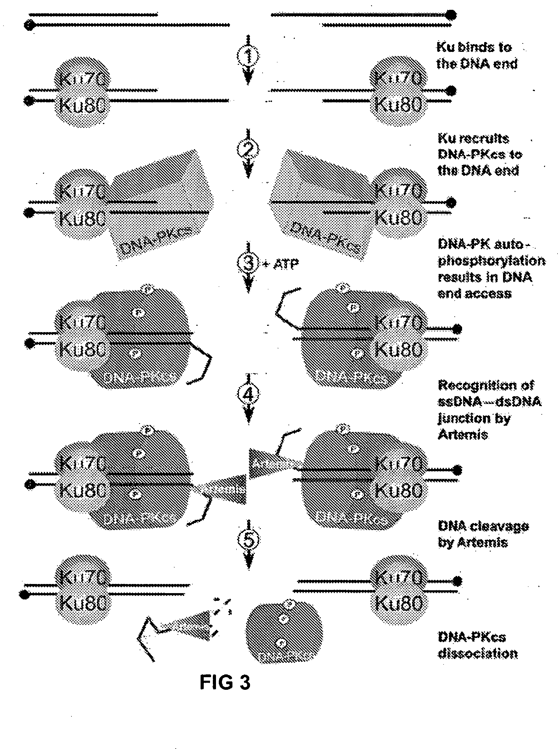

NHEJ is the dominant pathway of DSB repair and involves rejoining blunt ends or ends with short overhangs and begins with the recognition and juxtaposition of the broken ends. This is promoted by the DNA-PK complex consisting of the KU heterodimer (Ku70 and Ku80 [or Ku86]) and the DNA-PK catalytic subunit (DNA-PKcs). Maturation of the DSB ends is carried out by Artemis (Figure 3, from Goodarzi *et al.,* 2006) and resealing by the Xrcc4/DNA ligase IV complex. NHEJ is a relatively inaccurate process and is frequently accompanied by insertion and deletion of DNA sequence (Bleuyard et al., 2006, Goodarzi et al., 2006 The EMBO journal 25:3880-3889). Several genes are known to play a role in NHEJ, including KU70, KU80, and PARP-1.

Sequences from genes associated with the NHEJ pathway that can be used in the compositions used to alter the regulation of the pathway, such as the generation of the dsRNA are available from Public databases such as GeneBank entry AF283759.1 for AtKU70 and described herein elsewhere. The desired plant specific sequences can be identified by designing primers based on , for instance available Arabidopsis sequences, and subsequently identifying the desired orthologs.

### Homologous Recombination pathway

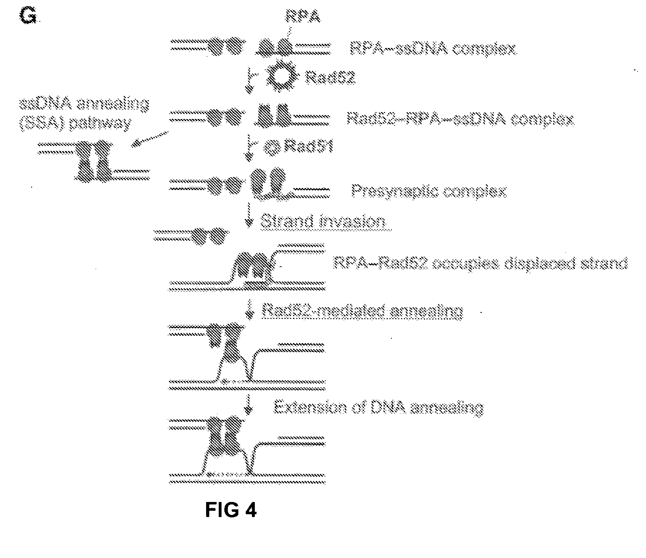

HR is an accurate repair process that uses the sister chromatid as.template and therefore ensures the fidelity of the repair. The first step towards HR repair is the resection of the DSBs to form single-stranded 3' overhangs. The ends processing is carried out by the MRN complex which consists of the Mre11, Rad50 and Nbs1 proteins. With the help of accessory proteins, Rad51 is recruited on the single-stranded ends and promotes the invasion of the homologous duplex (Figure 4 from Sugiyama et al., 2006 The EMBO journal, 1-10).

The captured strand is then extended by DNA synthesis and the second DSB end captured resulting in the formation of a double-Holliday which can be resolved by endonucleases, resulting in the formation of a crossover, or dissolved by the combined action of a helicase and a topoisomerase (Bleuyard et al, 2006; Branzei and Foiani, 2008).

In the current disclosure, the first transfection can be with a second composition that is capable of inducing double stranded DNA breaks. Examples are Zinc finger nucleases and Meganucleases (Cellectis, France), and TAL effector nucleases (Bosch et al (2009) Science 326: 1509 - 1512; Moscou et al. (2009) Science Vol 326: 1501). The Zinc finger nucleases are designed such using known technology that they preferably induce the double strand break at the desired position where second transfection, in certain embodiments relating to targeted mutagenesis's, intends to introduce the mutation from the mutagenic oligonucleotides. Zinc finger nucleases are proteins custom designed to cut at a certain DNA sequence. Zinc fingers domains comprise of approximately 30 amino acids which folds into a characteristic structure when stabilized by a zinc ion. The zinc finger domains are able to bind to DNA by inserting into the major groove of the DNA helix. Each zinc finger domain is able to bind to a specific DNA triplet (3 bps) via key amino acid residues at the α-helix region of the zinc finger. Thus, by changing these key amino acids, it is possible to alter the recognition specificity of a zinc finger for a certain triplet and thereby create a Zinc finger construct, deliberately aimed at a sequence of interest. The flexibility of the system is derived from the fact that the zinc finger domains can be joined together in series to bind to long DNA sequences. For instance, six zinc finger domains in series recognizes a specific 18 bps sequence which is long enough to be unique in a complex eukaryotic genome. A zinc finger nuclease (ZFN) is comprised of a series of zinc fingers fused to the nuclease Fokl. The ZFN is introduced into the cell, and will recognize and bind to a specific genomic sequence. As the Fokl nuclease cuts as a dimer, a second ZFN is required which recognizes a specific sequence on the opposite DNA strand at the cut site. A DNA cut, or double strand break (DSB) is then made in between the two targeted DNA sequences (Miller et al, 2007 Nature Biotech 25(7):778-785; Cathomen and Joung, 2008 Mol Ther 16(7):1200-1207; Foley et al., 2009 PLoS ONE 4(2):e4348). In the presence of a homologous sequence, which can either be the sister chromatid or a donor DNA construct, the DSB can be repaired by HR. This is the basis for the process of gene targeting whereby, rather than the sister chromatid being used for repair, information is copied from a donor construct that is introduced into the cell. The donor construct contains alterations compared with the original chromosomal locus, and thus the process of HR incorporates these alterations the genome.

The first transfection may comprise transfection with both the first and the second composition, simultaneously or sequentially (one after the other).

In the method according to the disclosure, a second transfection is performed to introduce the one or more molecules of interest.

The molecules of interest can be selected from the group consisting of chemicals, DNA, RNA, protein, oligonucleotides, and peptides. In certain embodiments, the molecule of interest is selected from amongst dsRNA, miRNA, siRNA, plasmids, mutagenic oligonucleotides, more preferably mutagenic oligonucleotides.

As the molecule of interest plasmid can be used that codes for a ZFN construct. The second transfection step then introduces a ZFN construct, which, upon expression, can induce DSBs that can be used in footprinting.

In certain embodiments, mutagenic oligonucleotides can be used as the molecule of interest. The mutagenic oligonucleotide, once transfected into the protoplast is capable of providing an alteration in the DNA of the protoplast. Preferably, the target DNA for the mutagenic oligonucleotide is from nuclear DNA. Alternatively, chloroplast or mitochondrial DNA can be used. In principle any mutagenic oligonucleotides described thus far in the art, such as RNA/DNA chimeric oligonucleotides, oligonucleotides including those containing LNAs, phosphorothioates, propyne-substitutions etc. can be used.

The use of a mutagenic oligonucleotide as the molecule of interest thus provides for a oligonucleotide mediated targeted nucleotide exchange (ODTNE)

### Oligonucleotide-mediated targeted nucleotide exchange (ODTNE)

Oligonucleotide-mediated targeted nucleotide exchange (ODTNE) refers to the use of single stranded oligonucleotides to correct or alter genomic loci by introducing mutation(s), such as single point mutations or deletions/insertions, therefore restoring the original gene function. This concept is the basis of gene therapy and personalized medicine and is extensively studied worldwide (Parekh-Olmedo et al., 2002, Neuron 33:495-498; Madsen et al., 2008 PNAS 105:10, 3909-3914; Leclerc et al, 2009 BMC Biotechnology 9:35, 1-16). Several parameters influencing the efficacy and efficiency of ODTNE have been identified and while some still require validation, it is well established now that a functional MMR system counteracts ODTNE (Igoucheva et al, 2008 Oligonucleotides 18:111-122; Kennedy Maguire and Kmiec, 2007 Gene 386:107-114; Papaioannou et al., 2009 J. Gene Med. 11:267-274). The use of ODTNE and the structure and design of the oligonucleotides that are functional in this technology are well described, *inter alia* in WO98/54330, WO99/25853, WO01/24615, WO01/25460, WO2007/084294, WO2007073149, WO2007073166, WO2007073170, WO2009002150. Based on the structural features of the mutagenic oligonucleotides disclosed herein and sequence information from the target sequence (gene to be altered) the skilled man can design the desired mutagenic oligonucleotide to be used in the second transfection step. The mutagenic oligonucleotides used in the present invention have a length that is in line with other mutagenic oligonucleotides used in the art, i.e. typically between 10-60 nucleotides, preferably 20-55 nucleotides, more preferably 25-50 nucleotides.

The present invention using a mutagenic oligonucleotide can be used for instance for altering a cell, correcting a mutation by restoration to wild type, inducing a mutation, inactivating an enzyme by disruption of coding region, modifying bioactivity of an enzyme by altering coding region, modifying a protein by disrupting the coding region, modifying miRNA targets, modifying precursor genes and many more purposes.

In certain variants of the disclosure, the molecule of interest is a DNA construct. A DNA construct is a DNA sequence that contains the sequence information of which it is desired that it is introduced in the cell (gene targeting). The DNA construct can be a ZFN construct.

Transfection, both the first and the second transfection can be achieved using the methods described in the art such as electroporation, biolistics, PEG-mediated transfection etc. There is a preference for PEG-mediated transfection. Conventional transfection such as PEG-mediated transfection (preferred) or biolistics can be carried out using state of the art methods (Sporlein et al (1991) Theor. Appl. Genet. 82, 712-722;Mathur and Koncz. Methods in Molecular Biology. Vol. 82: Arabidopsis protocols. J. Marinez-Zapater and J. Salinas Eds. Humana Press Inc. Totowa NJ.;Golds et al (1993) Bio/Technology 11, 95-100.).

Gene targeting is an extremely powerful technique which has many applications in both medicine and agriculture. It allows the precise manipulation of the genome, enabling biologists to study and exploit gene function. However, the efficiency of HR in nearly all cell types is low as it relies on the presence of a DSB in the chromosomal locus. The usefulness of ZFN's is thus their ability to induce a DSB at any chromosomal locus, and have been used to improve the efficiency of gene targeting a 100 fold. Once a DSB is produced, it can be repaired by either the NHEJ or the HR pathway. The efficiency of HR, and thus gene targeting, can be enhanced by inhibiting the NHEJ pathway so that the DSB's can be repaired by HR. This has been shown to indeed be the case in human and fungal cells (Fattah et al. 2008 Proc.Natl.Acad.Sci.USA 105:8703-8708; Meyer et al. 2007 J.Biotechnology 128:770-775; Bertolini et al. 2009 Mol.Biotechnol. 41: 106-114). The choice between NHEJ and HR may also depend on the cell cycle phases, in G1, NHEJ predominates due to the absence of homologous template while HR is more active in G2/M where a homologous sister chromatid is present (Branzei and Foiani, 2008, Nature reviews molecular biology).

### ODTNE and ZFN in plant breeding

Plant breeding uses natural genetic variation to improve plant performances by conventional crossing. However, natural variation is limited and many years required for a breeding program to produce a valuable new variety. Genetic variation can be created artificially and traditionally, this is done by chemical mutagenesis which introduces many mutations in the genome of the host plant. A few mutations will eventually give the phenotype of interest and can be used in a breeding program. These methods however have shortcomings such as the need for many backcrosses to eliminate residual mutations and the limited scope of mutations introduced by such chemicals. Technologies such as ODTNE and ZFN therefore represent attractive solutions to introduce genetic variation in a directed and clean way in plants. However, translating an animal system into a plant system represents quite a challenge, especially to replicate the physiological conditions known to promote targeted gene alteration.

A functional MMR system counteracts ODTNE and substantial increases in gene repair have been observed after knocking out MSH2 using siRNA. The methods however make use of a stably integrated siRNA construct and therefore MSH2 is constitutively suppressed which is not favorable since, in the long term, the resulting mutator phenotype will lead to the death of the plant. ODTNE has also been shown to be promoted in cells accumulating in the S phase of the cell cycle.

A method for transient suppression of specific mRNA in plant protoplasts has been described (An et al. 2003 Biosci.Biotechnol.Biochem. 67: 2674-2677) and it has now been found this may be a valuable tool for transient suppression of (endogenous) MMR genes in plants. Accumulation of cells in S phase is readily achievable using chemicals such as hydroxyurea or aphidicolin. The inventors have found that the coordination of these various parameters with the delivery of the oligonucleotide may potentate the effect of each individual parameter. To achieve this, the present invention provides MMR suppression while the cells are accumulating in the S phase of the cell cycle followed by the introduction of the oligonucleotide to drive the correction of the gene of interest.

The same holds for gene targeting where prior to introducing the donor construct, an increased proportion of cells in the S/G2/M phases of the cell cycle is desirable, NHEJ is suppressed, ZFN are expressed and DSBs generated.

In plant cells, introduction of foreign molecules in the cell is not as straightforward as in animal cells because of the presence of a very thick cell that needs to be removed for the molecule of interest to reach the protoplast. This is achieved by enzymatic digestion of the cell wall with cellulolytic and pectolytic enzymes, but as soon as the enzyme mixture is washed away, the cell will start reforming a cell wall. It is therefore critical to prevent cell wall reformation if one wants to retain the transformability of the protoplast over long periods of time, for example for at least 10, 30, 60 minutes, or 1, 2, 4, 6,8, 10, 12, 16, or 24 hours, or more; for example from 10 minutes to 24 hours.. Conveniently, chemicals exist that affect cell wall synthesis and can be used to maintain the protoplast naked until transfected with the various molecules of interest. In the present application, we provide evidence that the use of such cell wall inhibitors allows the sequential introduction of foreign molecules in plant protoplasts leading to improved efficiencies of oligonucleotide-mediated targeted gene alteration and gene targeting using ZFN.

Thus, in certain embodiments of the invention, to prevent reformation of the cell wall, a non-enzymatic composition is added to the protoplast culture. By disrupting, preventing, reducing and/or delaying cell wall reformation until the cells reach an appropriate stage in the cell cycle; more foreign molecules can be delivered to the cell, and an increase in the efficiency of transfection can be achieved. Removal of the non-enzymatic composition, for instance by washing or replacing the medium with a medium that does not contain the compound that inhibits the reformation of the cell wall allows the cell wall to from and the cell to continue the cell cycle.

The non-enzymatic composition can be added to the plant cell protoplast depending on the particular circumstances of the desired transfections. The composition can be added
- before or simultaneous with the first transfection;
- between the first and second transfection,
- before or simultaneous with the second transfection, or
after the second transfection.

The non-enzymatic composition that inhibits or prevents the formation of cell wall can be removed:
- before or simultaneous with the first transfection,
- between the first and second transfection,
- before or simultaneous with the second transfection, or
- after the second transfection and before the cell wall is allowed to form.

In this way, the reformation of the cell wall can be inhibited taking into account the desired transfection. For example, for the footprint formation at the tomato ALS locus as illustrated in Figure 5 , the composition is added before the first transfection step. In other examples (see Figure 6 and Figure 7), the composition is added (nearly) simultaneously with the first transfection. It is likewise possible to allow reformation of the cell wall for a brief period of time (1- 24 hours) and then stop further formation of the cell wall prior to the first transfection.

Time periods between the first transfection and the second transfection can vary from at least 10, 30, 60 minutes, or 1, 2, 4, 6, 8, 10, 12, 16, 24 hours, to several days, for example to 96 hours, or even more.. Typically the period is from 1 hour to 72 hours, preferably from 2 to 48 hours, more preferably from 4 to 42 hours, even more preferably between 12 and 36 hours.

Interfering with cell wall (re)formation (via inhibition, disruption, delay and/or reduction) is achieved by adding one or more chemical (i.e. non-enzymatic) compounds to the protoplast culture medium that, for instance, inhibit cellulose deposition or capture nascent cellulose microfibrils thus preventing their incorporation into an organized cell wall (Parekh-Olmedo et al (2003) Ann. NY Acad. Sci. 1002, 43-56; Anderson et al (2002) J. Plant Physiol. 159, 61-67;Meyer and Herth (1978) Chemical inhibition of cell wall formation and cytokinesis, but not of nuclear division, in protoplasts of Nicotiana tabacum L. cultivated in vitro. Plant 142(3), 253-262).

The chemical compounds that are used in the present invention are referred to in this application as 'cell wall formation inhibitors'. These chemical compounds are capable of preventing, disrupting, inhibiting and/or delaying the formation of the cellulose cell wall, indicated herein as 'inhibiting with cell wall formation'.

The protoplast culture may be allowed to go through its normal developmental cycle, only in absence of, or at least with a reduction in the formation of the cell wall. As the protoplast has gone through its developmental cycle and has come to the phase at which it is desired that the DNA synthesis commences, the cell wall formation inhibitor can be substantially removed from the protoplast culture, for instance by washing or by replacement of the culture medium.

Thus, the treatment of protoplasts with the cell wall formation inhibitors prohibits cell wall formation for, for example, at least 12-60 hours, or 24-48 hours, from the moment the inhibitor(s) is (are) added. Thus inhibiting cell wall formation for a sufficient period allows the use of conventional transfection technologies at a time in the cell cycle where the cell is normally not receptive for transfection. The use of the inhibitor typically does not influence the progression of the cell cycle.

The chemical under consideration should preferably prevent cell wall reformation without interfering significantly with cell cycle progression or being deleterious to the protoplasts at the concentration used. In this context, 'without interfering significantly' means that the chemical allows the cell cycle progression to continue for at least 50 %, at least 75%, preferably 85%, more preferably 95% of its normal rate, i.e. in absence of the chemical. In this context 'being deleterious' means that at least 50 %, at least 75%, preferably 85%, more preferably 95% of the protoplasts are not affected by the chemical in any other way than the inhibition of the cell wall reformation as described herein. /esp

Various chemicals interfere with cell wall formation. Many of those chemicals are commonly used as herbicides. For example, 2,6-dichlorobenzonitrile (DCB) (DeBolt et al (2007) Plant Physiology 145, 334-338;Anderson et al (2002) J. Plant Physiol. 159, 61-67.) is a well know herbicide that acts by inhibiting cellulose synthases therefore disrupting cell plate formation (Vaughn et al (1996) Protoplasma 194, 117-132). DCB has been shown to inhibit the motility of the cellulose synthase complexes without affecting their delivery to the plasma membrane (DeBolt et al (2007) Plant Physiology 145, 334-338). Furthermore, preferred cell wall formation inhibitors do not affect cell cycle progression (Galbraith and Shields (1982) The effect of inhibitors of cell wall synthesis on tobacco protoplast development. Physiologia Plantarum 55(1), 25-30;Meyer and Herth (1978) Chemical inhibition of cell wall formation and cytokinesis, but not of nuclear division, in protoplasts of Nicotiana tabacum L. cultivated in vitro. Plant 142(3), 253-262), or only to a limited extent as the cell cycle progression is of course of importance with respect to the present technology. DCB does not limit cell cycle progression and as such is a preferred cell wall formation inhibitor.

Other chemicals include the herbicide isoxaben (DeBolt et al (2007) Plant Physiology 145, 334-338), which inhibits integration of the cellulose synthase complexes in the plasma membrane and disrupts existing ones. Thus, in a preferred embodiment the cellulose synthesis inhibitor is a cellulose synthase inhibitor. In another embodiment, the chemical interferes with the genes responsible for cellulose synthesis, such as the CESA genes. Calcofluor white, also called fluorescent brightener, competes with cellulose microfibrils preventing their integration into a coordinated network (Roncero and Duran (1985) Journal of Bacteriology 163(3), 1180-1185, Haigler et al (1980) Science 210(4472), 903-906).

Other cell wall formation inhibitors are for instance cellulose biosynthesis inhibitors such as nitrile, benzamide and/or triazolocarboxamides herbicides, microtubule assembly inhibitors such as dinitroaniline, phosphoroamidate, pyridine, benzamide and/or benzenedicarboxylic acid herbicides and/or inhibitors of cellulose deposition.

In certain embodiments, the cellulose biosynthesis inhibitor is selected from the group consisting of dichlobenil, chlorthiamid, flupoxam, triazofenamide, phtoxazolin A, Phtoramycin, thaxtomin A, brefeldin A.

In certain embodiments, the microtubule assembly inhibitor, is selected from the group consisting of cobtorin, dinitroaniline, benefin (benfluralin), butralin, dinitramine, ethalfluralin, oryzalin, pendimethalin, trifluralin, amiprophos-methyl, butamiphos dithiopyr, thiazopyr propyzamide = pronamide, tebutam DCPA (chlorthal-dimethyl).

In certain embodiments, the inhibitor of cellulose deposition is quinclorac.

In certain embodiments, the cell wall formation inhibitor is selected from the group consisting of morlin (7-ethoxy-4-methyl chromen-2-one), isoxaben (CAS 82558-50-7, *N*-[3-(1-ethyl-1-methylpropyl)-1,2-oxazol-5-yl]-2,6-dimethoxybenzamide), AE F150944 (N2-(1-ethyl-3-phenylpropyl)-6-(1-fluoro-1-methylethyl)-1,3,5,-triazine-2,4-diamine), diclobenil (dichlorobenzonitrile), calcofluor and/or calcofluor white (4,4'-bis((4-anilino-6-bis(2-hydroxyethyl)amino-s-triazin-2-yl) amino)-, 2,2'-stilbenedisulfonic acid and salts thereof), oryzalin (CASRN - 19044-88-3, 4-(Dipropylamino)-3,5-dinitrobenzenesulfonamide), 5-tert-butyl-carbamoyloxy-3-(3-trifluromethyl) phenyl-4-thiazolidinone, coumarin, 3,4 dehydroproline, 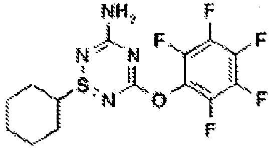 cobtorin, dinitroanitine, benefin (benfluralin), butralin, dinitramine, ethalfluralin, pendimethalin, trifluralin, amiprophos-methyl, butamiphos dithiopyr, thiazopyr propyzamide = pronamide, tebutam, DCPA (chlorthal-dimethyl), quinclorac.

In certain embodiments, mixtures of two or more of the above listed chemicals can be used. These can be added to the protoplast sample simultaneously or in succession.

The amount and concentration of the non-enzymatic composition will differ between the various (mixtures of) chemicals and protoplast systems but can be readily determined by the skilled man, based on the available literature cited herein, together with some initial basic experimentation.

The plant cell may be a dicot or a monocot.

Preferred dicots in this respect are selected from the group consisting of Magnoliaceae, Ranunculaceae, Cactaceae, Asteraceae, Fagaceae, Solanaceae, Brassicaceae, Lamiaceae, Rosaceae, Oleaceae, Cucurbitaceae, and Umbelifereae.

Preferred monocots in this respect are selected from the group consisting of Poaceae, Orchidaceae, Iridaceae, Lemnaceae, Liliaceae, and Alliaceae.

Preferred crops are potato, maize, tomato, tobacco, cotton, soy, rapeseed.

Freshly isolated protoplasts are usually naturally synchronized in G0 (Galbraith and Shields (1982). Physiologia Plantarum 55(1), 25-30). Depending on the desired transfection and the desired cell phase (S-phase, the M-phase, the G1 and/or G2 phase), the need for extra synchronization of the protoplasts may be advantageous in certain embodiments to further enhance efficiency of the overall process or of the transfection step. Different protoplasts, such as derived from mesophyll, meristem, or cell suspension may or may not be actively diving and synchronization of the cell phase may be desirable to achieve adequate transfection.

Thus in certain embodiments, the method further comprises a step of synchronizing the cell phase of the plant cell or plant cell protoplast.

The synchronization of the cell phase can be achieved by nutrient deprivation such as phosphate starvation, nitrate starvation, ion starvation, serum starvation, sucrose starvation, auxin starvation. Synchronization can also be achieved by adding a synchronizing agent to the protoplast sample.

The synchronization can take place:
- before the plant cell protoplast is formed from the plant cell; or
- before the first transfection; or
- before the second transfection; or
- between the first and the second transfection;

The synchronization step may also contain a step in which the synchronizing agent is removed, for instance by washing or replacement of the medium.
- before the plant cell protoplast is formed from the plant cell; or
- before the first transfection; or
- before the second transfection; or
- between the first and the second transfection; or
- after or simultaneous with the second transfection.

The synchronizing step may be performed independently (such as before, after or simultaneously with) of the step of contacting the plant cell protoplast with a non-enzymatic composition that inhibits or prevents the (re)formation of the cell wall.

Thus, in certain embodiments, a synchronizing agent can be added to the protoplast sample. Synchronizing agents such as aphidocolin (preferred), hydroxyurea (preferred), thymidine, colchicine, cobtorin, dinitroaniline, benefin (benfluralin), butralin, dinitramine, ethalfluralin, oryzalin, pendimethalin, trifluralin, amiprophos-methyl, butamiphos dithiopyr, thiazopyr propyzamide = pronamide, tebutam DCPA (chlorthal-dimethyl), mimosine, anisomycin, alpha amanitin, lovastatin, jasmonic acid, abscisic acid, menadione, cryptogeine, heat, hydrogenperoxide, sodiumpermanganate, indomethacin, epoxomycin, lactacystein, icrf 193, olomoucine, roscovitine, bohemine, staurosporine, K252a, okadaic acid, endothal, caffeine,MG132, cycline dependent kinases and cycline dependent kinase inhibitors as well as their target mechanism, the amounts and concentrations and their associated cell cycle phase are described for instance in "Flow Cytometry with plant cells", J. Dolezel c.s. Eds. Wiley-VCH Verlag 2007 pp 327 ff. There exists a preference for aphidicolin and/or hydroxyurea

In preferred embodiments of the method of the present invention, directed at footprint formation at a selected locus, the method comprises the steps of cell wall digestion to generate protoplasts, cell wall inhibition by a composition comprising a cell wall formation inhibitor (preferably DCB), addition of a synchronizing agent (preferably hydroxyurea) (at the same time or prior to the first transfection), addition of a dsRNA against KU70 (first composition), addition (preferably after a period of, for example, about 6, 12, 18 or 24 hours) of a ZFN construct (second composition or second transfection), removal of the synchronizing agent simultaneously with or just before the second transfection).

In preferred embodiments, aimed at gene targeting events, the method according to the invention comprises the formation of plant cell protoplasts, addition of cell wall formation inhibitor, addition of synchronizing agent, ZFN construct and/or dsRNA against, for instance but not restricted to, KU70 (NHEJ) (first transfection) and after a period of synchronization of for example, 6, 12, 18 or 24 hours, a second transfection of a donor construct with removal of the synchronization agent.

In preferred embodiments aimed at ODTNE in protoplasts, the plant cells are provided with a synchronising agent up to 48 hours before protoplast formation. After cell wall digestion, the cell wall inhibitor is added together with dsRNA against MMR (MSH2 or other MMR-related genes) (first transfection). At the desired cell cycle phase, the cell wall inhibition is lifted, the synchronization agent removed, the mutagenic oligonucleotide added for the second transfection and the cell allowed to continue the cell cycle.

The disclosure also pertains to kits for transfecting plant cell protoplasts comprising two or more selected from the group consisting of a first composition, a second composition, a non-enzymatic composition that inhibits or prevents the formation of the cell wall, a synchronizing agent and one or more foreign molecules of interest.

### Brief description of the Figures

Figure 1: A schematic representation for signaling downstream MMR following mismatch recognition.
Figure 2: A schematic representation for NHEJ and HR, taken from Branzei and Foiani, 2008 - 8(9):1038-46.
Figure 3: A schematic representation of the maturation of DSB ends.
Figure 4: A schematic representation of Homologous recombination.

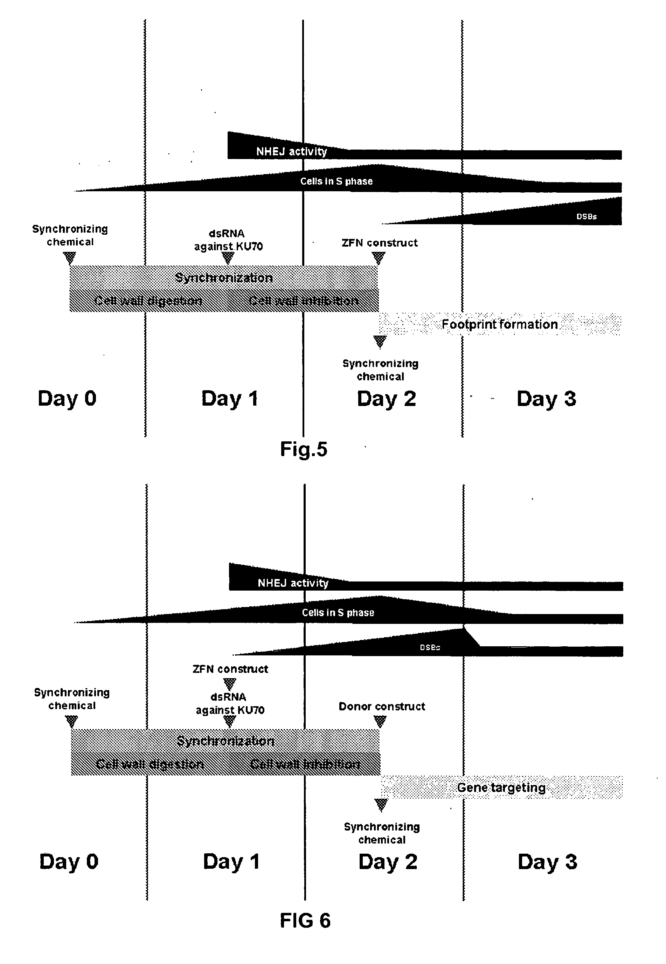

Figure 5: Experimental design for footprint formation in plant protoplasts.
Figure 6: Experimental design for gene targeteing events.

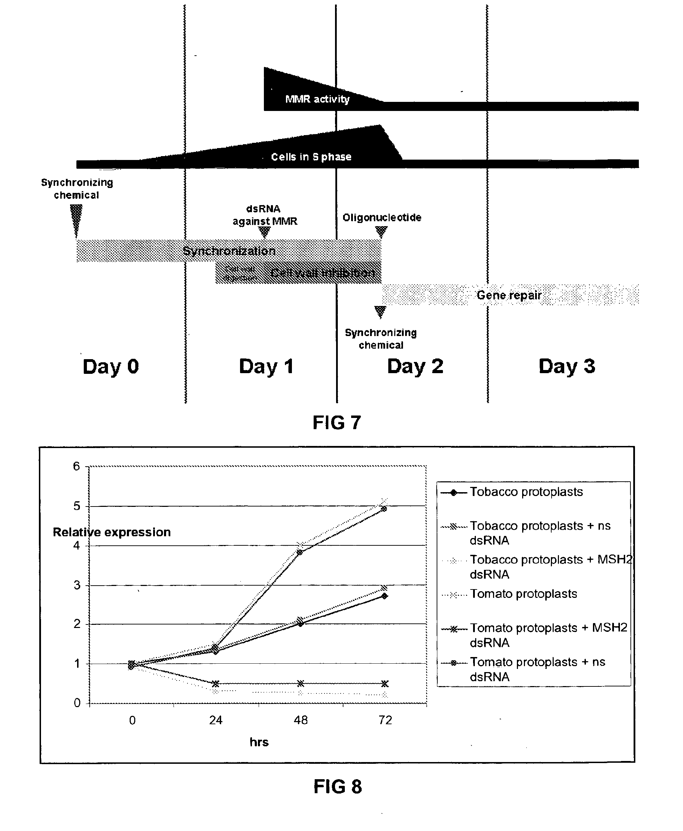

Figure 7: Experimental deign for meGFP restoration in BY-2 protoplasts
Figure 8: Levels of MSH2 in tobacco and tomato protoplasts upon addition of dsRNA

### Examples:

### Plant Mismatch Repair genes and non-homologous end joining genes

The public databases were screened for tobacco and tomato EST's sharing homology with genes involved in the MMR pathway (MSH2) and the NHEJ pathway (Ku70). The regions used to produce dsRNA are underlined, dsRNA was produced according to protocols well known in the art. In addition, a non-specific dsRNA species was generated derived from a plasmid which shows no significant homology with any of the genes of interest. This was used as a control to demonstrate that the presence of dsRNA *per se* is not responsible for suppression of specific mRNA's.

### Tomato Ku70

### Tobacco MSH2

### Assessment of NtMSH2 and LeKu70 down-regulation

Twenty four hours after transfection of protoplasts with dsRNA against LeKu70 or MilliQ water, total RNA was isolated using the RNAeasy Kit (Qiagen). cDNA synthesis was performed using the Quantitect RT kit (Qiagen). Levels of endogenous LeKu70 were measured using a Light Cycler apparatus (Roche). The primers used for mRNA quantification are listed below.

| Gene | Forward primer | SEQ ID NO | Reverse primer | SEQ ID NO |
|---|---|---|---|---|
| Tomato Ku70 | ACCAGCTTAGGAAACGCA | 3 | AGCACCAGTATCAGCACA | 4 |
| Tobacco MSH2 | CACACATTGACCCTTCTAATCGC | 5 | AGAAATCCTCCAACTCAGATGCC | 6 |

### Tomato protoplast isolation

In vitro shoot cultures of the tomato M82 cultivar are maintained on MS20 medium supplemented with 0.8% Micro-Agar with a 16/8 h photoperiod of 2000 lux at 25°C and 60-70% RH. One gram of young leaves is gently sliced in CPW9M and transferred to the enzyme solution (CPW9M containing 2% cellulose onozuka RS, 0.4% macerozyme onozuka R10, 2.4-D (2mg/ml), NAA (2mg/ml), BAP (2mg/ml) pH5.8), and hydroxyurea (2mM)). Digestion is allowed to proceed overnight at 25°C in the dark. The next morning, Petri dishes are gently swirled for one hour to release protoplast. The protoplast suspension is filtered through a 50 µm mesh stainless steel sieve and protoplasts harvested by centrifugation at room temperature for 5 min. at 85xg. The protoplast pellet is re-suspended into CPW9M supplemented with 2 mM hydroxyurea and 3 mL of CPW18S are added to the bottom of each tube. Live protoplasts that accumulate at the interface between the two layers during centrifugation (10 minutes, room temperature, 85xg) are collected and their density evaluated using and a haemocytometer. Protoplasts are harvested by centrifugation for 5 min at 85x g at room temperature and re-suspended in MaMg medium supplemented with 2 mM hydroxyurea to a final density of 10⁶ per mL.

### Tomato protoplast transfection

### Footprint formation (Example 1)

For each transfection, 250000 protoplasts are mixed with 25 µg of double-stranded RNA against tomato Ku70 and 250 µL of PEG-Solution (40% PEG4000 (Fluka #81240), 0.1M Ca(NO₃)₂, 0.4M mannitol). Transfection is allowed to proceed for 20 minutes at room temperature. Five mL of 0.275M Ca(NO₃)₂ are added dropwise and thoroughly mixed in. Transfected protoplasts are harvested by centrifugation for 5 minutes at 85xg at room temperature and washed twice in CPW9M. Finally, protoplasts are re-suspended in K8p supplemented with 2mg.L⁻¹ dichlobenil and 2 mM hydroxyurea to a final density of 250000 per mL and incubate overnight at 25°C in the dark. The next morning protoplasts are harvested by centrifugation at 85xg for 5 minutes at room temperature, washed once in CPW9M supplemented with 2mM hydroxyurea and live protoplasts are isolated as described above. Live protoplasts are re-suspended in MaMg to a final density of 10⁶ per mL and transfected as described above with 20 µg of ZFN construct (Townsend et al. 2009 Nature)*.* Protoplasts are then embedded in alginate and cultivated in K8p culture medium.

### Gene targeting (Example 2)

For each transfection, 250000 protoplasts are mixed with 25 µg of double-stranded RNA against tomato Ku70; 20 µg of ZFN construct (Townsend et al. 2009 Nature) and 250 µL of PEG-Solution (40% PEG4000 (Fluka #81240), 0.1M Ca(NO₃)₂, 0.4M mannitol). Transfection is allowed to proceed for 20 minutes at room temperature. Five mL of 0.275M Ca(NO₃)₂ are added dropwise and thoroughly mixed in. Transfected protoplasts are harvested by centrifugation for 5 minutes at 85xg at room temperature and washed twice in CPW9M. Finally, protoplasts are re-suspended in K8p supplemented with 2mg.L⁻¹ dichlobenil and 2 mM hydroxyurea to a final density of 250000 per mL and incubate overnight at 25°C in the dark. The next morning protoplasts are harvested by centrifugation at 85xg for 5 minutes at room temperature, washed once in CPW9M supplemented with 2mM hydroxyurea and live protoplasts are isolated as described above. Live protoplasts are re-suspended in MaMg to a final density of 10⁶ per mL and transfected as described above with 20 µg of donor construct. Protoplasts are then embedded in alginate and cultivated in K8p culture medium.

### Detection of footprints (Example 1)

After 3 days of cultivation, alginate disks are dissolved in sodium citrate, protoplasts harvested by centrifugation and frozen in liquid nitrogen for subsequent DNA extraction using the DNAeasy kit (Qiagen). The full length ALS open reading frame is amplified by PCR using proof reading Taq polymerase, the PCR product cloned into the TOPO XL PCR cloning vector (Invitrogen) and transformed to E. Coli One Shot TOP10 competent cells (Invitrogen). Bacteria are plated on LB agar supplemented with 100 µg.mL⁻¹ carbenicillin and incubated overnight at 37°C. The next morning, 400 individual clones are picked up and used for high resolution melting curve analysis on a Light Cycler apparatus (Roche) to identify clones with a mismatch at the ALS locus. Positive clones are confirmed by sequencing.

### Detection of gene targeting events (Example 2)

After 14 days of cultivation, alginate disks are cut into 5 mm strips and placed on the surface of TM-DB medium solidified with 0.8% micro agar and supplemented with 20 nM chlorsulfuron. Calli resulting from a gene targeting event will be resistant to chlorsulfuron and will develop in 6-8 weeks. Resistant calli are sampled, DNA extracted using Qiagen Plant DNA easy kit. The full length coding sequence of the ALS gene is amplified by PCR and the presence of mutations confirmed by sequencing.

### EXAMPLE 3

### Plant cell lines

A tobacco Bright Yellow 2 cell suspension containing a non-functional EGFP gene was produced by introducing a point mutation in the chromophore region of the protein resulting in the formation of a premature stop codon. This line is used as reporter system to test the influence of various parameters on the repair of the EGFP gene by oligonucleotide-mediated targeted gene repair.

cDNA sequence of the mutated EGFP (mEGFP) the position of the mutation is indicated in underlined and Bold (G to T).

### Repairing and control oligonucleotide sequences

| | | |
|---|---|---|
| GFP 7 | SEQ ID NO 8 | T*G*A*A*CAGCTCCTCGCCCTTGC*T*C*A*C |
| GFP 8 | SEQ ID NO 9 | T*G*A*A*CAGCTCCTAGCCCTTGC*T*C*A*C |

| | | |
|---|---|---|
| * indicate phosphorothioate modifications | | |

### Tobacco protoplast isolation

Five mL of a 7d-old tobacco Bright Yellow 2 (BY-2) cell suspension culture weekly maintained in BY-2 culture medium (Nagata *et al.* 1999 *Method Cell Sci*) are transferred to a 50 mL Erlenmeyer flask containing 45 mL of BY-2 culture medium supplemented with 2 mM hydroxyurea. Cells are allowed to divide for 24 hours and harvested by centrifugation at 1000 rpm for 10 minutes at room temperature. To the packed cell volume, 25 mL of BY-2 enzyme mixture (1% (w/v) cellulase Onozuka RS, 0.05% pectinase Y23, 0.2% driselase from *Basidiomycetes* sp) in MDE (0.25 g KCI, 1.0 g MgSO₄.7H₂O, 0.136 g of KH₂PO₄, 2.5 g polyvinylpyrrolidone (MW 10,000), 6 mg naphthalene acetic acid and 2 mg 6-benzylaminopurine in a total volume of 900 ml. The osmolality of the solution is adjusted to 600 mOsm.kg⁻¹ with sorbitol, the pH to 5.7) are added. Cells are transferred to a TC quality Petri dish and digestion is allowed to proceed for 4 hours at 25°C under gentle agitation (40 rpm). The protoplast suspension is filter through a 50 µm mesh stainless steel sieve and harvested by centrifugation at 800 rpm for 5 minutes at 5°C. Protoplasts are re-suspended into ice-cold KC wash medium (0.2% CaCl₂.2H₂O, 1.7% KCl, 540 mOsm.Kg⁻¹ with KCI, pH 5.7) supplemented with 2 mM hydroxyurea and centrifuged at 800 rpm for 5 minutes at 5°C. Protoplasts are re-suspended in KC wash medium supplemented with 2 mM hydroxyurea and 3 mL of CPW18S are added to the bottom of each tube. Live protoplasts will accumulate at the interface of the two media during centrifugation at 800 rpm for 10 minutes at 5°C. Live protoplasts are harvested and their density evaluated using a haemocytometer. Protoplast density is adjusted to 10⁶ per mL using ice-cold KC wash medium.

### Tobacco protoplasts transfection

Tobacco protoplasts transfection is performed as for tomato protoplasts. Tobacco protoplasts are transfected with 12.5 µg of dsRNA against tobacco MSH2. Transfected protoplasts are re-suspended in 2.5 mL To culture medium supplemented with 2 mM hydroxyurea and 2 mg.L⁻¹ dichlobenil. To culture medium contained (per liter, pH 5.7) 950 mg KNO₃, 825 mg NH₄NO₃, 220 mg CaCl₂.2H₂O, 185 mg MgSO₄.7H₂O, 85 mg KH₂PO₄, 27.85 mg FeSO₄.7H₂O, 37.25 mg Na₂EDTA.2H₂O, the micro-nutrients according to Heller's medium (Heller, R. 1953 Ann Sci Nat Bot Biol Veg), vitamins according to Morel and Wetmore's medium (Morel, G. and R.H. Wetmore 1951 Amer. J. Bot.), 2% (w/v) sucrose, 3 mg naphthalene acetic acid, 1 mg 6-benzylaminopurine and a quantity of mannitol to bring the osmolality to 540 mOsm.kg⁻¹ and transferred to a 35 mm Petri dish. The next day, protoplasts are harvested by centrifugation and washed with ice-cold KC wash medium supplemented with 2mM hydroxyurea and 2 mg.L⁻¹ dichlobenil. Live protoplasts are harvested and transfected as described above with 1.6 nmol of oligonucleotides complementary to the transcribed strand and containing (GFP 7) or not (GFP 8) one mismatch with the targeted sequence. Oligonucleotides are protected from nuclease degradation by 4 phosphorothioate linkages on both the 3' and 5' ends. Protoplasts are finally re-suspended into To culture medium without hydroxyurea or dichlobenil. After 24 hours, EGFP restoration is scored using a Nikon Eclipse TS100-F equipped with band pass GFP filter cube and fitted with a CFI Super Plan Fluor ELWD 20XC objective.

### Results

### Down regulation of tobacco and tomato MSH2

Results are given in Figure 8. The results demonstrate that the level of MSH mRNA increases after isolation. The majority of leaf protoplasts are derived from mesophyll cells which are not actively dividing. After isolation, the hormones in the medium induce re-entry of the cell into the cell cycle and a consequent induction of the levels of MMR genes. Addition of a non-specific dsRNA (sharing no homology with MSH2) does not affect the expression levels whereas MSH2 dsRNA is effective at reducing MSH2 mRNA levels to 5-20% of that found in protoplasts upon isolation. We found similar results for the dsRNA targeted to both MLH1 and KU70.

### Footprint formation at the tomato ALS locus (Example 1, Figure 5)

All samples were treated with 2 mM hydroxyurea (see material and methods)

| **Transfected at Day 1 with:** | **Transfected at Day 2 with:** | **Unique footprints** |
|---|---|---|
| - | - | 0 |
| dsRNA against Ku70 | - | 0 |
| - | ZFN construct | 0 |
| Overnight treatment-with 2 mg.L⁻¹ dichlobenil | - | 0 |
| Overnight treatment with 2 mg.L⁻¹ dichlobenil | ZFN construct | 13 |
| dsRNA against Ku70 + overnight treatment with 2 mg.L⁻¹ dichlobenil | - | 0 |
| dsRNA against Ku70 + overnight treatment with 2 mg.L⁻¹ dichlobenil | ZFN construct | 53 |

### Gene targeting events at the tomato ALS locus (Example 2, Figure 6)

### Example 2: experimental design for efficient gene targeting in plant protoplasts, see Figure 6.

All samples were treated with 2 mM hydroxyurea (see material and methods)

| **Transfected at Day 1 with:** | **Transfected at Day 2 with:** | **Resistant calli (%)** |
|---|---|---|
| - | - | 0 |
| dsRNA against Ku70 + ZFN construct | - | 0 |
| dsRNA against Ku70 + ZFN construct | Donor construct | 0 |
| dsRNA against Ku70 + ZFN construct + overnight treatment with 2 mg.L⁻¹ dichlobenil | - | 0 |
| dsRNA against Ku70 + ZFN construct + donor construct | - | 0.02 |
| dsRNA against Ku70 + ZFN construct + overnight treatment with 2 mg.L⁻¹ dichlobenil | Donor construct | 3.4 |

### meGFP restoration in BY-2 protoplasts

### Example 3: experimental design for efficient ODTNE in plant protoplasts, See Figure 7 All samples were treated with 2 mM hydroxyurea (see material and methods)

| **Transfected at Day 1 with:** | **Transfected at Day 2 with:** | **GFP positive protoplasts after 24 hours (/10⁶)** |
|---|---|---|
| - | - | 0 |
| repairing oligonucleotide | - | 0 |
| - | repairing oligonucleotide | 0 |
| overnight treatment with 2 mg.L⁻¹ dichlobenil | repairing oligonucleotide | 2 |
| MSH2 dsRNA | repairing oligonucleotide | 0 |
| MSH2 dsRNA + overnight treatment with 2 mg.L⁻¹ dichlobenil | repairing oligonucleotide | 122 |
| MSH2 dsRNA + overnight treatment with 2 mg.L⁻¹ dichlobenil | oligonucleotide w/o mismatch | 0 |

From the examples above, it is clear that optimization of the sequence of events required for footprint formation, gene targeting or ODTNE by means of cell wall inhibition leads to substantial improvements in all the described processes.

## Claims

1. Method for the introduction of one or more molecules of interest in a plant cell protoplast comprising the steps of
- providing the plant cell protoplast by enzymatically degrading and/or removing the cell wall from a plant cell;
- performing a first transfection of the plant cell protoplast with
i. a first composition that is capable of altering the regulation of one or more pathways selected from the group consisting of Mismatch Repair System or Non-Homologous End Joining
- performing a second transfection of the plant cell protoplast with one or more molecules of interest, wherein the one or more molecules of interest is/are selected from the group consisting of oligonucleotides or mutagenic oligonucleotides;
- allowing the cell wall to form;
wherein the second transfection is performed after the first transfection, wherein the method further comprises contacting the plant cell protoplast with a non-enzymatic composition that inhibits or prevents the (re)formation of the cell wall
- before or simultaneous with the first transfection; or
- between the first and second transfection, or
- before or simultaneous with the second transfection
and the method further comprises the step of removing the non-enzymatic composition that inhibits or prevents the formation of cell wall
- before or simultaneous with the first transfection, or
- between the first and second transfection, or
- before or simultaneous with the second transfection, or
- after the second transfection,
and before the cell wall is allowed to form.

2. Method according to claim 1, wherein the mutagenic oligonucleotide has a length of between 10 - 60 nucleotides.

3. Method according to any of the previous claims, wherein the altering of the regulation is down-regulation of one or more of the pathways, preferably transient down-regulation of the pathway.

4. Method according to any of the previous claims, further comprising a step of synchronizing the cell cycle phase of the plant cell or plant cell protoplast.

5. Method according to claim 4, wherein the synchronization is achieved by contacting the plant cell or plant cell protoplast with a synchronizing agent, preferably
- before, or simultaneous with, the plant cell protoplast is formed from the plant cell; or
- before or simultaneous with the first transfection; or
- before or simultaneous with the second transfection; or
- between the first and the second transfection.

6. Method according to claim 5 wherein the method further comprises a step of removing the synchronizing agent
- before the plant cell protoplast is formed from the plant cell; or
- before or simultaneous with the first transfection; or
- before or simultaneous with the second transfection; or
- between the first and the second transfection; or
- after or simultaneous with the second transfection.

7. Method according to any of the claims 4 - 6, wherein the synchronizing step is performed independently (such as before, after or simultaneously with) of the step of contacting the plant cell protoplast with a non-enzymatic composition that inhibits or prevents the (re)formation of the cell wall.

8. Method according to claim 1, wherein the non-enzymatic composition that inhibits the formation of cell walls contain one or more cell wall formation inhibitors selected from the group consisting of
a. cellulose biosynthesis inhibitor;
b. microtubule assembly inhibitor;
c. inhibitor of cellulose deposition;
d. other cell wall formation inhibitor.

9. Method according to claim 8, wherein the cellulose biosynthesis inhibitor is selected from the group consisting of dichlobenil, chlorthiamid, flupoxam, triazofenamide, phtoxazolin A, Phtoramycin, thaxtomin A, brefeldin A.

10. Method according to claim 8, wherein the microtubule assembly inhibitor, is selected from the group consisting of cobtorin, dinitroaniline, benefin (benfluralin), butralin, dinitramine, ethalfluralin, oryzalin, pendimethalin, trifluralin, amiprophos-methyl, butamiphos dithiopyr, thiazopyr propyzamide = pronamide, tebutam DCPA (chlorthal-dimethyl).

11. Method according to claim 8, wherein the inhibitor of cellulose deposition is quinclorac.

12. Method according to claim 8, wherein the other cell wall formation inhibitor is selected from the group consisting of morlin (7-ethoxy-4-methyl chromen-2-one), isoxaben (CAS 82558-50-7, *N*-[3-(1-ethyl-1-methylpropyl)-1,2-oxazol-5-yl]-2,6-dimethoxybenzamide), AE F150944 (N2-(1-ethyl-3-phenylpropyl)-6-(1-fluoro-1-methylethyl)-1,3,5,-triazine-2,4-diamine), Dichlobenil (dichlorobenzonitrile), calcofluor and/or calcofluor white (4,4'-bis((4-anilino-6-bis(2-hydroxyethyl)amino-s-triazin-2-yl) amino)-, 2,2'-stilbenedisulfonic acid and salts thereof), oryzalin (CASRN - 19044-88-3, 4-(Dipropylamino)-3,5-dinitrobenzenesulfonamide), 5-tert-butyl-carbamoyloxy-3-(3-trifluromethyl) phenyl-4-thiazolidinone, coumarin, 3,4 dehydroproline, 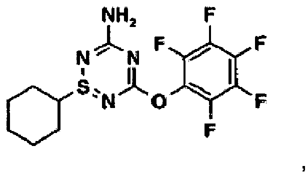 cobtorin, dinitroaniline, benefin (benfluralin), butralin, dinitramine, ethalfluralin, pendimethalin, trifluralin, amiprophos-methyl, butamiphos dithiopyr, thiazopyr, propyzamide = pronamide, tebutam, DCPA (chlorthal-dimethyl), quinclorac.

13. Method according to any of the previous claims, wherein the first composition is capable of altering the regulation of one or more of MutS, MutL, MutH, MSH2, MSH3, MSH6, MSH7, MLH1, MLH2, MLH3, PMS1, the DNA-PK complex Ku70, Ku80, Ku86, Mre11, Rad50, RAD51, XRCC4, Nbs1, PARP-1.

14. Method according to any of the previous claims, wherein the first composition comprises a dsRNA.

15. Method according to claim 4 - 7, wherein the synchronization of the cell cycle phase synchronizes the protoplast in the S-phase, the M-phase, the G1 and/or G2 phase of the cell cycle.

16. Method according to claim 4, 7, or 15, wherein the synchronization of the cell cycle phase is achieved by nutrient deprivation such as phosphate starvation, nitrate starvation, ion starvation, serum starvation, sucrose starvation, auxin starvation.

17. Method according to claim 5, wherein the synchronizing agent is selected from one or more of the group consisting of aphidicolin, hydroxyurea, thymidine, colchicine, cobtorin, dinitroaniline, benefin (benfluralin), butralin, dinitramine, ethalfluralin, oryzalin, pendimethalin, trifluralin, amiprophos-methyl, butamiphos dithiopyr, thiazopyr propyzamide = pronamide, tebutam DCPA (chlorthal-dimethyl), mimosine, anisomycin, alpha amanitin, lovastatin, jasmonic acid, abscisic acid, menadione, cryptogeine, heat, hydrogenperoxide, sodiumpermanganate, indomethacin, epoxomycin, lactacystein, icrf 193, olomoucine, roscovitine, bohemine, staurosporine, K252a, okadaic acid, endothal, caffeine,MG132, cycline dependent kinases and cycline dependent kinase inhibitors.

18. Method according to any of the claim 1 -17, wherein the time period between the first transfection and the second transfection is at least 10, 30, 60 minutes, 1, 2, 4, 6, 8, 10, 12, 16, or 24 hours, but preferably less than 96 hours, preferably the period is from 1 hour to 72 hours, more preferably from 2 to 48 hours, even more preferably from 4 to 42 hours, still even more preferably between 12 and 36 hours.

19. Method according to any of the previous claims, wherein the time period between the first transfection and the second transfection is at least 10, 30, 60 minutes, 1, 2, 4, 6, 8, 10, 12, 16, or 24 hours, but preferably less than 96 hours, preferably the period is from 1 hour to 72 hours, more preferably from 2 to 48 hours, even more preferably from 4 to 42 hours, still even more preferably between 12 and 36 hours and wherein the method further comprises contacting the plant cell protoplast with a non-enzymatic composition that inhibits or prevents the (re)formation of the cell wall
- before or simultaneous with the first transfection; or
- between the first and second transfection, or
- before or simultaneous with the second transfection,
and the method further comprises the step of removing the non-enzymatic composition that inhibits or prevents the formation of cell wall
- before or simultaneous with the first transfection, or
- between the first and second transfection, or
- before or simultaneous with the second transfection, or
- after the second transfection,
and before the cell wall is allowed to form.

## Patentansprüche

1. Verfahren zur Einführung von einem oder mehreren Molekülen von Interesse in einen Pflanzenzellprotoplasten, umfassend die Schritte
- den Pflanzenzellprotoplasten durch enzymatisches Verdauen und/oder Entfernen der Zellwand aus einer Pflanzenzelle bereitzustellen;
- eine erste Transfektion des Pflanzenzellprotoplasten mit
i. einer ersten Zusammensetzung, die fähig ist, die Regulation von einem oder mehreren Wegen, ausgewählt aus der Gruppe, bestehend aus dem Mismatch Repair System oder Non-Homologous End Joining, durchzuführen,
- eine zweite Transfektion des Pflanzenzellprotoplasten mit einem oder mehreren Molekülen von Interesse durchzuführen, wobei das eine oder die mehreren Moleküle von Interesse aus der Gruppe ausgewählt ist/sind, bestehend aus Oligonucleotiden oder mutagenen Oligonucleotiden;
- die Zellwand sich bilden zu lassen;
wobei die zweite Transfektion nach der ersten Transfektion durchgeführt wird, wobei das Verfahren ferner umfasst, den Pflanzenzellprotoplasten mit einer nicht-enzymatischen Zusammensetzung in Kontakt zu bringen, welche die (Neu-)Bildung der Zellwand inhibiert oder verhindert,
- vor oder gleichzeitig mit der ersten Transfektion oder
- zwischen der ersten und zweiten Transfektion oder
- vor oder gleichzeitig mit der zweiten Transfektion,
und das Verfahren ferner den Schritt umfasst, die nicht-enzymatische Zusammensetzung, welche die Bildung der Zellwand inhibiert oder verhindert,
- vor oder gleichzeitig mit der ersten Transfektion oder
- zwischen der ersten und zweiten Transfektion oder
- vor oder gleichzeitig mit der zweiten Transfektion oder
- nach der zweiten Transfektion,
und bevor man die Zellwand sich bilden lässt, zu entfernen.

2. Verfahren gemäß Anspruch 1, wobei das mutagene Oligonucleotid eine Länge von zwischen 10 - 60 Nucleotide aufweist.

3. Verfahren gemäß einem der vorhergehenden Ansprüche, wobei es sich beim Ändern der Regulation um Herunterregulierung von einem oder mehreren der Wege handelt, vorzugsweise um vorübergehende Herunterregulierung des Weges.

4. Verfahren gemäß einem der vorhergehenden Ansprüche, ferner umfassend einen im Synchronisieren der Zellzyklusphase der Pflanzenzelle oder des Pflanzenzellprotoplasten bestehenden Schritt.

5. Verfahren gemäß Anspruch 4, wobei die Synchronisierung durch In-Kontakt-Bringen der Pflanzenzelle oder des Pflanzenzellprotoplasten mit einem synchronisierenden Mittel erreicht wird, vorzugsweise
- bevor der Pflanzenzellprotoplast aus der Pflanzenzelle gebildet wird oder gleichzeitig damit oder
- vor oder gleichzeitig mit der ersten Transfektion oder
- vor oder gleichzeitig mit der zweiten Transfektion oder
- zwischen der ersten und der zweiten Transfektion.

6. Verfahren gemäß Anspruch 5, wobei das Verfahren ferner einen Schritt umfasst, bestehend im Entfernen des sychronisierenden Mittels
- bevor der Pflanzenzellprotoplast aus der Pflanzenzelle gebildet wird oder
- vor oder gleichzeitig mit der ersten Transfektion oder
- vor oder gleichzeitig mit der zweiten Transfektion oder
- zwischen der ersten und der zweiten Transfektion oder
- nach oder gleichzeitig mit der zweiten Transfektion.

7. Verfahren gemäß einem der Ansprüche 4 - 6, wobei der synchronisierende Schritt unabhängig (wie zum Beispiel vorher, danach oder gleichzeitig damit) von dem Schritt, bestehend im In-Kontakt-Bringen des Pflanzenzellprotoplasten mit einer nicht-enzymatischen Zusammensetzung, welche die (Neu-)Bildung der Zellwand inhibiert oder verhindert, durchgeführt wird.

8. Verfahren gemäß Anspruch 1, wobei die nicht-enzymatische Zusammensetzung, welche die Bildung von Zellwänden inhibiert, einen oder mehrere Zellwandbildungs-Inhibitoren enthält, ausgewählt aus der Gruppe, bestehend aus
a. einem Cellulosebiosynthese-Inhibitor;
b. einem Inhibitor der Zusammenlagerung von Mikrotubuli;
c. einem Inhibitor der Celluloseablagerung;
d. einem anderen Zellwandbildungs-Inhibitor.

9. Verfahren gemäß Anspruch 8, wobei der Cellulosebiosynthese-Inhibitor aus der Gruppe ausgewählt ist, bestehend aus Dichlobenil, Chlorthiamid, Flupoxam, Triazofenamid, Phtoxazolin A, Phtoramycin, Thaxtomin A, Brefeldin A.

10. Verfahren gemäß Anspruch 8, wobei der Inhibitor der Zusammenlagerung von Mikrotubuli aus der Gruppe ausgewählt ist, bestehend aus Cobtorin, Dinitroanilin, Benefin (Benfluralin), Butralin, Dinitramin, Ethalfluralin, Oryzalin, Pendimethalin, Trifluralin, Amiprophos-methyl, Butamiphos Dithiopyr, Thiazopyr Propyzamid = Pronamid, Tebutam DCPA (Chlorthal-dimethyl).

11. Verfahren gemäß Anspruch 8, wobei es sich bei dem Inhibitor der Celluloseablagerung um Quinclorac handelt.

12. Verfahren gemäß Anspruch 8, wobei der andere Zellwandbildungs-Inhibitor aus der Gruppe ausgewählt ist, bestehend aus Morlin (7-Ethoxy-4-methylchromen-2-on), Isoxaben (CAS 82558-50-7, *N*-[3-(1-Ethyl-1-methylpropyl)-1,2-oxazol-5-yl]-2,6-dimethoxybenzamid), AE F150944 (N2-(1-Ethyl-3-phenylpropyl)-6-(1-fluor-1-methylethyl)-1,3,5,-triazin-2,4-diamin), Dichlobenil (Dichlorbenzonitril), Calcofluor und/oder Calcofluor Weiß (4,4'-Bis((4-anilino-6-bis(2-hydroxyethyl)amino-s-triazin-2-yl)amino)-,2,2'-stilbendisulfonsäure und deren Salze), Oryzalin (CASRN - 19044-88-3, 4-(Dipropylamino)-3,5-dinitrobenzolsulfonamid), 5-Tert-butyl-carbamoyloxy-3-(3-trifluormethyl)-phenyl-4-thiazolidinon, Cumarin, 3,4-Dehydroprolin, 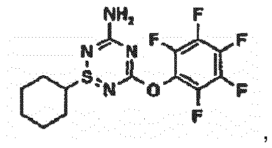 Cobtorin, Dinitroanilin, Benefin (Benfluralin), Butralin, Dinitramin, Ethalfluralin, Pendimethalin, Trifluralin, Amiprophos-methyl, Butamiphos Dithiopyr, Thiazopyr, Propyzamid = Pronamid, Tebutam, DCPA (Chlorthal-dimethyl), Quinclorac.

13. Verfahren gemäß einem der vorhergehenden Ansprüche, wobei die erste Zusammensetzung fähig ist, die Regulation von einem oder mehreren aus MutS, MutL, MutH, MSH2, MSH3, MSH6, MSH7, MLH1, MLH2, MLH3, PMS1, dem DNA-PK Komplex Ku70, Ku80, Ku86, Mre11, Rad50, RAD51, XRCC4, Nbs1, PARP-1, zu ändern.

14. Verfahren gemäß einem der vorhergehenden Ansprüche, wobei die erste Zusammensetzung eine dsRNA umfasst.

15. Verfahren gemäß Anspruch 4 - 7, wobei die Synchronisierung der Zellzyklusphase den Protoplasten in der S-Phase, der M-Phase, der G1- und/oder G2-Phase des Zellzyklus synchronisiert.

16. Verfahren gemäß Anspruch 4, 7 oder 15, wobei die Synchronisierung der Zellzyklusphase durch Nährstoffentzug, wie zum Beispiel Phosphatverarmung, Nitratverarmung, Ionenverarmung, Serumverarmung, Saccharoseverarmung, Auxinverarmung, erreicht wird.

17. Verfahren gemäß Anspruch 5, wobei das synchronisierende Mittel aus einem oder mehreren aus der Gruppe ausgewählt ist, bestehend aus Aphidicolin, Hydroxyharnstoff, Thymidin, Colchicin, Cobtorin, Dinitroanilin, Benefin (Benfluralin), Butralin, Dinitramin, Ethalfluralin, Oryzalin, Pendimethalin, Trifluralin, Amiprophos-methyl, Butamiphos Dithiopyr, Thiazopyr Propyzamid = Pronamid, Tebutam DCPA (Chlorthal-dimethyl), Mimosin, Anisomycin, alpha-Amanitin, Lovastatin, Jasmonsäure, Abszisinsäure, Menadion, Cryptogein, Wärme, Wasserstoffperoxid, Natriumpermanganat, Indomethacin, Epoxomycin, Lactacystein, Icrf 193, Olomoucine, Roscovitin, Bohemine, Staurosporin, K252a, Okadainsäure, Endothal, Koffein, MG132, Cyclin-abhängigen Kinasen und Cyclin-abhängigen Kinaseinhibitoren.

18. Verfahren gemäß einem der Ansprüche 1 - 17, wobei der Zeitraum zwischen der ersten Transfektion und der zweiten Transfektion mindestens 10, 30, 60 Minuten, 1, 2, 4, 6, 8, 10, 12, 16 oder 24 Stunden, aber vorzugsweise weniger als 96 Stunden beträgt, vorzugsweise beträgt der Zeitraum von 1 Stunde bis 72 Stunden, stärker bevorzugt von 2 bis 48 Stunden, noch stärker bevorzugt von 4 bis 42 Stunden, noch stärker bevorzugt zwischen 12 und 36 Stunden.

19. Verfahren gemäß einem der vorhergehenden Ansprüche, wobei der Zeitraum zwischen der ersten Transfektion und der zweiten Transfektion mindestens 10, 30, 60 Minuten, 1, 2, 4, 6, 8, 10, 12, 16 oder 24 Stunden, aber vorzugsweise weniger als 96 Stunden beträgt, vorzugsweise beträgt der Zeitraum von 1 Stunde bis 72 Stunden, stärker bevorzugt von 2 bis 48 Stunden, noch stärker bevorzugt von 4 bis 42 Stunden, noch stärker bevorzugt zwischen 12 und 36 Stunden und wobei das Verfahren ferner umfasst, den Pflanzenzellprotoplasten mit einer nicht-enzymatischen Zusammensetzung in Kontakt zu bringen, welche die (Neu-)Bildung der Zellwand inhibiert oder verhindert,
- vor oder gleichzeitig mit der ersten Transfektion oder
- zwischen der ersten und zweiten Transfektion oder
- vor oder gleichzeitig mit der zweiten Transfektion,
und das Verfahren ferner den Schritt umfasst, bestehend im Entfernen der nicht-enzymatischen Zusammensetzung, welche die (Neu-)Bildung der Zellwand inhibiert oder verhindert,
- vor oder gleichzeitig mit der ersten Transfektion oder
- zwischen der ersten und zweiten Transfektion oder
- vor oder gleichzeitig mit der zweiten Transfektion oder
- nach der zweiten Transfektion,
und bevor man die Zellwand sich bilden lässt.

## Revendications

1. Procédé pour l'introduction d'une ou de plusieurs molécules d'intérêt dans un protoplaste de cellule végétale, comprenant les étapes de
- fourniture du protoplaste de cellule végétale par dégradation et/ou élimination par voie enzymatique de la paroi cellulaire d'une cellule végétale ;
- réalisation d'une première transfection du protoplaste de cellule végétale avec
i. une première composition qui est capable de modifier la régulation d'une ou de plusieurs voies choisies dans le groupe constitué par le système de réparation des mésappariements ou la jonction non homologue des extrémités ('Non-Homologous End Joining')
- réalisation d'une deuxième transfection du protoplaste de cellule végétale avec une ou plusieurs molécules d'intérêt, dans laquelle les une ou plusieurs molécules d'intérêt est/sont choisie(s) dans le groupe constitué par les oligonucléotides ou les oligonucléotides mutagènes ;
- permettre à la paroi cellulaire de se former ;
dans lequel la deuxième transfection est réalisée après la première transfection, dans lequel le procédé comprend en outre la mise en contact du protoplaste de cellule végétale avec une composition non-enzymatique qui inhibe ou empêche la (re)formation de la paroi cellulaire
- avant ou simultanément à la première transfection ; ou
- entre la première et la deuxième transfection, ou
- avant ou simultanément à la deuxième transfection
et le procédé comprend en outre l'étape d'élimination de la composition non enzymatique qui inhibe ou empêche la formation de la paroi cellulaire
- avant ou simultanément à la première transfection, ou
- entre la première et la deuxième transfection, ou
- avant ou simultanément à la deuxième transfection, ou
- après la deuxième transfection,
et avant que la paroi cellulaire soit permise de se former.

2. Procédé selon la revendication 1, dans lequel l'oligonucléotide mutagène a une longueur comprise entre 10 - 60 nucléotides.

3. Procédé selon l'une quelconque des revendications précédentes, dans lequel la modification de la régulation est une régulation négative d'une ou plusieurs des voies, de préférence une régulation négative transitoire de la voie.

4. Procédé selon l'une quelconque des revendications précédentes, comprenant en outre une étape de synchronisation de la phase du cycle cellulaire de la cellule végétale ou du protoplaste de cellule végétale.

5. Procédé selon la revendication 4, dans lequel la synchronisation est obtenue par la mise en contact de la cellule végétale ou du protoplaste de cellule végétale avec un agent de synchronisation, de préférence
- avant, ou simultanément avec, que le protoplaste de cellule végétale soit formé à partir de la cellule végétale ; ou
- avant ou simultanément à la première transfection ; ou
- avant ou simultanément à la deuxième transfection ; ou
- entre la première et la deuxième transfection.

6. Procédé selon la revendication 5, dans lequel le procédé comprend en outre une étape d'élimination de l'agent de synchronisation
- avant que le protoplaste de la cellule végétale soit formé à partir de la cellule végétale ; ou
- avant ou simultanément à la première transfection ; ou
- avant ou simultanément à la deuxième transfection ; ou
- entre la première et la deuxième transfection ; ou
- après ou simultanément à la deuxième transfection.

7. Procédé selon l'une quelconque des revendications 4 - 6, dans lequel l'étape de synchronisation est réalisée de façon indépendante (tel que avant, après ou simultanément avec) de l'étape de mise en contact du protoplaste de cellule végétale avec une composition non enzymatique qui inhibe ou empêche la (re)formation de la paroi cellulaire.

8. Procédé selon la revendication 1, dans lequel la composition non enzymatique qui inhibe la formation des parois cellulaires contient un ou plusieurs inhibiteurs de la formation de la paroi cellulaire choisis dans le groupe constitué par
a. un inhibiteur de la biosynthèse de la cellulose ;
b. un inhibiteur de l'assemblage des microtubules ;
c. un inhibiteur du dépôt de la cellulose ;
d. un autre inhibiteur de la formation de la paroi cellulaire.

9. Procédé selon la revendication 8, dans lequel l'inhibiteur de la biosynthèse de la cellulose est choisi dans le groupe constitué par le dichlobénil, le chlorthiamide, le flupoxam, le triazofénamide, la phtoxazoline A, la phtoramycine, la thaxtomine A, la bréfeldine A.

10. Procédé selon la revendication 8, dans lequel l'inhibiteur de l'assemblage des microtubules est choisi dans le groupe constitué par la cobtorine, la dinitroaniline, la bénéfine (benfluraline), la butraline, la dinitramine, l'éthalfluraline, l'oryzaline, la pendiméthaline, la trifluraline, l'amiprophos-méthyl, le butamiphos, le dithiopyr, le thiazopyr propyzamide = pronamide, le tébutame, le DCPA (chlorthal-diméthyl).

11. Procédé selon la revendication 8, dans lequel l'inhibiteur du dépôt de cellulose est le quinclorac.

12. Procédé selon la revendication 8, dans lequel l'inhibiteur de la formation de la paroi cellulaire est choisi dans le groupe constitué par le morlin (7-éthoxy-4-méthyl chromén-2-one), l'isoxaben (CAS 82558-50-7, *N*-[3-(1-éthyl-1-méthylpropyl)-1,2-oxazol-5-yl]-2,6-diméthoxybenzamide), l'AE F150944 (N2-(1-éthyl-3-phénylpropyl)-6-(1-fluoro-1-méthyléthyl)-1,3,5,-triazine-2,4-diamine), le dichlobénil (dichlorobenzonitrile), le calcofluor et/ou le calcofluor blanc (acide 4,4'-bis((4-anilino-6-bis(2-hydroxyéthyl)amino-s-triazin-2-yl)amino)-2,2'-stilbène-disulfonique et les sels de celui-ci), l'oryzaline (CASRN-19044-88-3, 4(dipropylamino)-3,5-dinitrobenzènesulfonamide), la 5-tert-butyl-carbamoyloxy-3-(3-trifluorométhyl)phényl-4-thiazolidinone, la coumarine, la 3,4-déshydroproline, 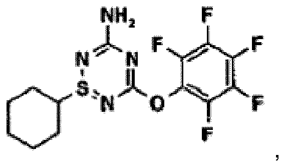 la cobtorine, la dinitroaniline, la bénéfine (benfluraline), la butraline, la dinitramine, l'éthalfluraline, la pendiméthaline, la trifluraline, l'amiprophos-méthyl, le butamiphos dithiopyr, le thiazopyr, le propyzamide = pronamide, le tébutame, le DCPA (chlorthal-diméthyl), le quinclorac.

13. Procédé selon l'une quelconque des revendications précédentes, dans lequel la première composition est capable de modifier la régulation d'un ou plusieurs parmi MutS, MutL, MutH, MSH2, MSH3, MSH6, MSH7, MLH1, MLH2, MLH3, PMS1, le complexe ADN-PK Ku70, Ku80, Ku86, Mre11, Rad50, RAD51, XRCC4, Nbs1, PARP-1.

14. Procédé selon l'une quelconque des revendications précédentes, dans lequel la première composition comprend un ARNdb ('dsRNA').

15. Procédé selon la revendication 4 - 7, dans lequel la synchronisation de la phase du cycle cellulaire synchronise le protoplaste dans la phase S, la phase M, la phase G1 et/ou G2 du cycle cellulaire.

16. Procédé selon la revendication 4, 7, ou 15, dans lequel la synchronisation de la phase du cycle cellulaire est obtenue par une privation de nutriments telle que la carence en phosphate, la carence en nitrate, la carence en ions, la carence en sérum, la carence en saccharose ('sucrose'), la carence en auxine.

17. Procédé selon la revendication 5, dans lequel l'agent de synchronisation est choisi parmi un ou plusieurs éléments du groupe constitué par l'aphidicoline, l'hydroxyurée, la thymidine, la colchicine, la cobtorine, la dinitroaniline, la bénéfine (benfluraline), la butraline, la dinitramine, l'éthalfluraline, l'oryzaline, la pendiméthaline, la trifluraline, l'amiprophos-méthyl, le butamiphos, le dithiopyr ('butamiphos dithiopyr'), le thiazopyr propyzamide = pronamide, le tébutame DCPA (chlorthal-diméthyl), la mimosine, l'anisomycine, l'alpha amanitine, la lovastatine, l'acide jasmonique, l'acide abscissique, la ménadione, la cryptogéine, la chaleur, le peroxyde d'hydrogène, le permanganate de sodium, l'indométacine, l'époxomycine, la lactacystéine, l'icrf 193, l'olomoucine, la roscovitine, la bohemine, la staurosporine, K252a, l'acide okadaïque, l'endothal, la caféine, MG132, les kinases cycline dépendantes et les inhibiteurs de kinases cycline dépendantes.

18. Procédé selon l'une quelconque des revendications 1 - 17, dans lequel la période entre la première transfection et la deuxième transfection est d'au moins 10, 30, 60 minutes, 1, 2, 4, 6, 8, 10, 12, 16, ou 24 heures, mais de préférence inférieure à 96 heures, de préférence la période est de 1 heure à 72 heures, plus préférablement de 2 à 48 heures, encore plus préférablement de 4 à 42 heures, encore plus préférablement entre 12 et 36 heures.

19. Procédé selon l'une quelconque des revendications précédentes, dans lequel la période entre la première transfection et la deuxième transfection est d'au moins 10, 30, 60 minutes, 1, 2, 4, 6, 8, 10, 12, 16, ou 24 heures, mais de préférence inférieure à 96 heures, de préférence la période est de 1 heure à 72 heures, plus préférablement de 2 à 48 heures, encore plus préférablement de 4 à 42 heures, encore plus préférablement entre 12 et 36 heures et dans lequel le procédé comprend en outre la mise en contact du protoplaste de cellule végétale avec une composition non enzymatique qui inhibe ou empêche la (re)formation de la paroi cellulaire
- avant ou simultanément à la première transfection ; ou
- entre la première et la deuxième transfection, ou
- avant ou simultanément à la deuxième transfection,
et le procédé comprend en outre l'étape d'élimination de la composition non enzymatique qui inhibe ou empêche la formation de la paroi cellulaire
- avant ou simultanément à la première transfection, ou
- entre la première et la deuxième transfection, ou
- avant ou simultanément à la deuxième transfection, ou
- après la deuxième transfection,
et avant que la paroi cellulaire soit permise de se former.
